Europäisches Patentamt
European Patent Office
Office européen des brevets

(19) (11) Veröffentlichungsnummer: **0 327 492 A1**

(12) # EUROPÄISCHE PATENTANMELDUNG

(21) Anmeldenummer: **89810031.8**

(22) Anmeldetag: **13.01.89**

(51) Int. Cl.[4]: **C 07 D 401/04**
C 07 D 471/14,
C 07 D 215/54,
C 07 D 221/04,
C 07 D 491/04,
C 07 D 471/04, A 01 N 43/50,
A 01 N 43/90
//(C07D471/14,235:00,221:00,
209:00),(C07D491/04,307:00,
221:00),(C07D471/04,221:00,
209:00)

(30) Priorität: **22.01.88 CH 226/88 06.12.88 CH 4513/88**

(43) Veröffentlichungstag der Anmeldung:
**09.08.89 Patentblatt 89/32**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Dürr, Dieter, Dr.**
**Brändelistalweg 16**
**CH-4103 Bottmingen (CH)**

**Szczepanski, Henry, Dr.**
**Bodenmatt**
**CH-4323 Wallbach (CH)**

**Brunner, Hans-Georg, Dr.**
**Wannenstrasse 14**
**CH-4415 Lausen (CH)**

Patentanspruch für folgenden Vertragsstaat: ES.

(54) **Herbizid und pflanzenwuchsregulatorisch wirksame Chinolin- und Pyrindinverbindungen.**

(57) Die vorliegende Erfindung betrifft neue herbizid und pflanzenwuchsregulatorisch wirksame Chinolin- und Pyrindinderivate, Verfahren zu deren Herstellung, Mittel, welche diese Verbindungen enthalten, neue Zwischenprodukte sowie die Verwendung der erfindungsgemässen Verbindungen als Selektivherbizide und Pflanzenwuchsregulatoren.

Die neuen Verbindungen entsprechen der Formel I

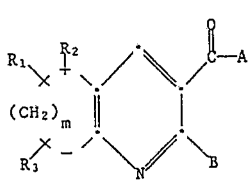

(I),

worin
$R_1$ $C_1$-$C_4$-Alkyl;
$R_2$ und $R_3$ unabhängig voneinander Wasserstoff; oder $C_1$-$C_4$-Alkyl;
$R_1$ und $R_2$ ausserdem gemeinsam einen geminal gebundenen $-(CH_2)_n-$Ring;
m 3 oder 4;
n 2 bis 5;
A OH, $O^{\ominus}M^{\oplus}$ oder $C_1$-$C_4$-Alkoxy;
$M^{\oplus}$ ein Kationäquivalent eines Alkali- oder Erdalkalimetalls oder einer stickstoffhaltigen Base;
B ein Imidazolon der Formel

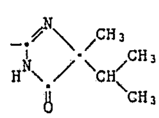

; oder

A und B gemeinsam ein anelliertes Imidazolon der Formel

EP 0 327 492 A1

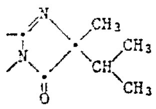

bedeutet,
sowie Salze von Verbindungen der Formel I mit Säuren, Basen und Komplexbildnern.

## Beschreibung

### Herbizid und pflanzenwuchsregulatorisch wirksame Chinolin- und Pyrindinverbindungen

Die vorliegende Erfindung betrifft neue herbizid und pflanzenwuchsregulatorisch wirksame Chinolin- und Pyrindinderivate, Verfahren zu deren Herstellung, Mittel, welche diese Verbindungen enthalten, neue Zwischenprodukte sowie die Verwendung der erfindungsgemässen Verbindungen als Herbizide.

Aus der EP-A-41 623 ist bekannt geworden, dass 2-(5-Isopropyl-5-methyl-1H-imidazol-4-on-2-yl)-pyridin- und -chinolin-3-carbonsäuren und deren Derivate herbizid wirksam sind.

Weiterhin ist aus der EP-A-95 105 und aus GB-A-2 174 395 bekannt geworden, dass 2-(6-Isopropyl-5-methyl-1H-imidazol-4-on-2-yl)-5,6,7,8-tetrahydrochinolin und -5,6-dihydro-pyrindin-3-carbonsäuren ebenfalls herbizid wirksam sind.

Diese Herbizidklasse wurde auch als Defoliationsmittel in Baumwollkulturen oder als Mittel zur Verbesserung der Verzweigung in Leguminosen vorgeschlagen (EP-A-41623 und GB-A-2174395). Zusätzlich beschreibt die EP-A-41624 die Verwendung dieser Stoffklasse zur Verbesserung der axilliaren Verzweigung, der Schösslings- und Blütenbildung, des Ertrags von landwirtschaftlichen und gartenbaulichen Nutzpflanzen sowie zur Beschleunigung des Reifungsprozesses von Getreidenutzpflanzen.

Die aus dem Stand der Technik bekannten Verbindungen befriedigen nicht immer im Hinblick auf Wirkungsstärke, Wirkungsspektrum, Wirkungsdauer oder Selektivität.

Gegenstand der Erfindung sind neue, im carbocyclisch anellierten Ring substituierte Tetrahydrochinolinderivate bzw. Dihydropyrindinderivate mit verbesserter Herbizidwirkung. Ausserdem sind die erfindungsgemässen Verbindungen pflanzenwuchsregulatorisch wirksam.

Die Erfindung betrifft die neuen Verbindungen der Formel I

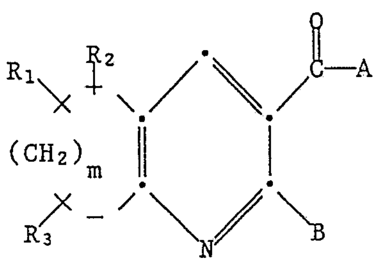

(I),

worin

$R_1$ $C_1$-$C_4$-Alkyl;

$R_2$ und $R_3$ unabhängig voneinander Wasserstoff; oder $C_1$-$C_4$-Alkyl;

$R_1$ und $R_2$ ausserdem gemeinsam einen geminal gebundenen $-(CH_2)_n-$ Ring;

m 3 oder 4;

n 2 bis 5;

A OH, $O^{\ominus}M^{\oplus}$ oder $C_1$-$C_4$-Alkoxy;

$M^{\oplus}$ ein Kationäquivalent eines Alkali- oder Erdalkalimetalls oder einer stickstoffhaltigen Base;

B ein Imidazolon der Formel

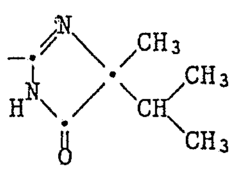

;

oder

A und B gemeinsam ein anelliertes Imidazolon der Formel

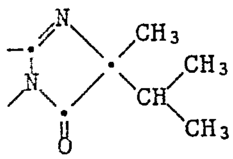

bedeutet,
sowie Salze von Verbindungen der Formel I mit Säuren, Basen und Komplexbildnern.

Die als mögliche Substituenten in Formel I angegebenen generischen Begriffe umfassen beispielsweise die nachstehend angegebenen spezifischen Einzelsubstituenten, wobei diese Aufzählung keine Einschränkung des Erfindungsgegenstandes darstellt.

$C_1$-$C_4$-Alkyl umfasst Methyl, Ethyl, n-Propyl, iso-Propyl, n-Butyl, sek-Butyl, iso-Butyl und tert.-Butyl.

$C_1$-$C_4$-Alkoxy umfasst Methoxy, Ethoxy, n-Propoxy, iso-Propyloxy, 1-Butyloxy, sek.-Butyloxy, iso-Butyloxy und tert.-Butyloxy.

Als Kation $M^{\oplus}$ kommen sowohl die Alkali- bzw. Erdalkalionen von Li, Na, K, Rb, Mg oder Ca in Frage, wie auch quarternäre oder protonierte stickstoffhaltige Basen wie $NH_4^{\oplus}$, $[N(H)_m(R)_n]^{\oplus}$ (mit $m+n=4$ ,und R = $C_1$-$C_6$-Alkyl, gegebenenfalls substituiert durch Hydroxy, oder $C_1$-$C_6$-Alkoxy), oder heterocyclische Basen wie Morpholin oder Piperidin. Zu nennen sind unter anderem $NH(C_2H_5)_3^{\oplus}$ oder $NH(CH_2CH_2OH)_3^{\oplus}$.

Die Verbindungen der Formel I können in unterschiedlichen isomeren und tautomeren Formen vorliegen. Diesen Isomeren bzw. Tautomeren, wie auch die Kombinationen unterschiedlicher Stereoisomere werden ebenfalls von Formel I umfasst.

Die Substituenten $R_1$ und $R_2$ können erfindungsgemäss einen geminal gebundenen $-(CH_2)_n-$Ring mit n = 2 bis 5 bedeuten. Sie bilden somit zusammen mit dem anellierten carbocyclischen $-(CH_2)_n-$Ring ein spirocyclisches System, in dem die Reste $R_1$ und $R_2$ gemeinsam für spirocyclisch gebundene 3-, 4-, 5- oder 6-Ringe stehen. Bevorzugt sind 3-, 5- und 6-Ringe. Besonders bevorzugt sind 3-Ringe.

Definitionsgemäss können die Reste A und B in Formel I für einen über die Positionen 1 und 2 anellierten Imidazol-5-on-Rest stehen. Zudem ist B als einfach gebundener Imidazolon-Rest definiert. Formel I umfasst somit die nachstehend angegebenen Verbindungen der Formel Ia, Ib und Ic.

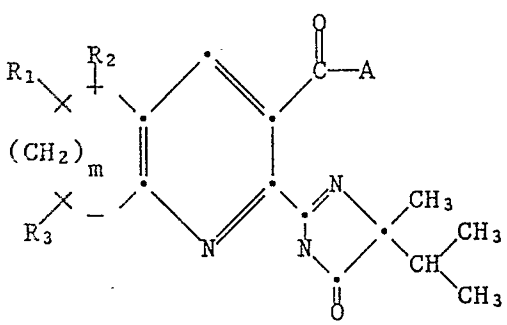

(Ia)

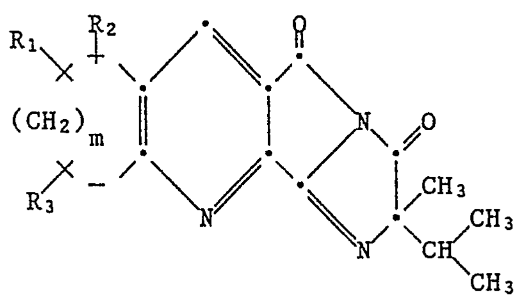

(Ib)

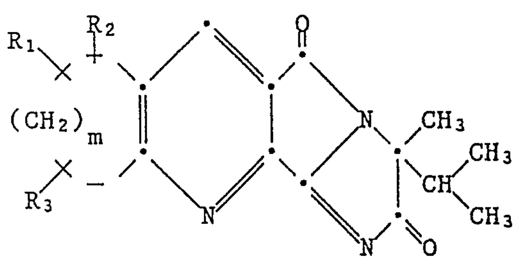

(Ic)

Da in dem in (I) anellierten Carbocyclus m die Werte 3 und 4 annehmen kann, liegen den erfindungsgemässen Verbindungen ein Dihydropyrindin-(m = 3) oder Tetrahydrochinolin-System (m = 4) als Stammsystem zugrunde.

Die zur Beschreibung der Bindungsstellen für die Substituenten $R_1$ bis $R_3$ in den Tetracyclen Ib und Ic

verwendete Numerierung ist für die Dihydropyrindinreihe

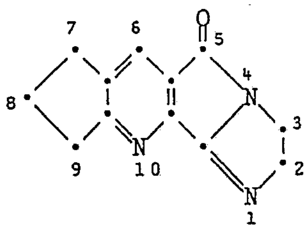

und für die Tetrahydrochinolinreihe:

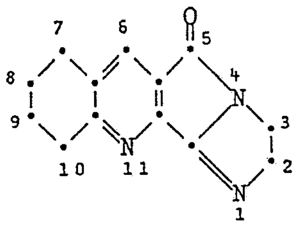

Bevorzugt sind die Verbindungen der Formel I,
worin
$R_1$ $C_1$-$C_3$-Alkyl;
$R_2$ und $R_3$ unabhängig voneinander Wasserstoff oder $C_1$-$C_3$-Alkyl;
$R_1$ und $R_2$ ausserdem gemeinsam einen geminal gebundenen $-(CH_2)_n-$Ring;
m 3 oder 4;
n 2, 4 oder 5;
A OH, $O^{\ominus}M^{\oplus}$ oder $C_1$-$C_3$-Alkoxy;
B ein Imidazolon der Formel

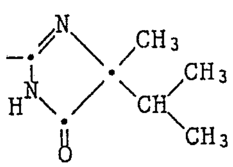 ;

oder
A und B gemeinsam ein anelliertes Imidazolon der Formel

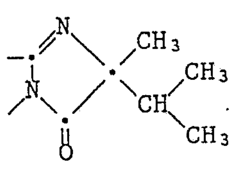

bedeutet.

Besonders bevorzugt sind die Verbindungen der Formel I,
worin
$R_1$ Methyl oder Ethyl;
$R_2$ und $R_3$ unabhängig voneinander Wasserstoff; Methyl oder Ethyl;
$R_1$ und $R_2$ ausserdem gemeinsam einen geminal gebundenen $-(CH_2)_n-$Ring mit $n = 2$ oder 4

bedeutet und
die Reste m, A und B wie zuvor definiert sind.

Insbesondere betrifft die Erfindung Verbindungen der Formel I,
worin
$R_1$ Methyl;
$R_2$ und $R_3$ unabhängig voneinander Methyl oder Wasserstoff;
$R_1$ und $R_2$ ausserdem gemeinsam einen geminal gebundenen $-(CH_2)_n-$Ring mit $n = 2$
bedeutet und
die Reste m, A und B wie zuvor definiert sind.

Weiterhin bevorzugt sind die Verbindungen der Formel Ia

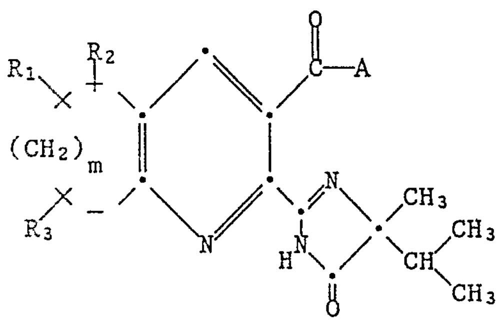

(Ia)

in der $R_1$, $R_2$, $R_3$, A und m wie zuvor definiert sind.

Ein weiterer Einfindungsgegenstand sind die heterocyclisch anellierten Verbindungen der Formel Ib

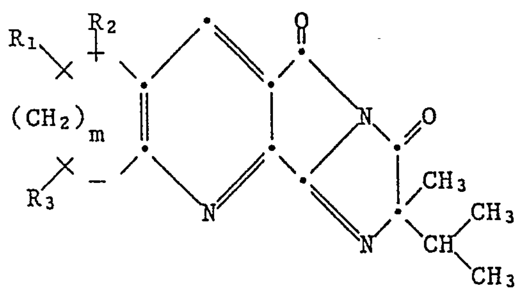

(Ib)

und Ic

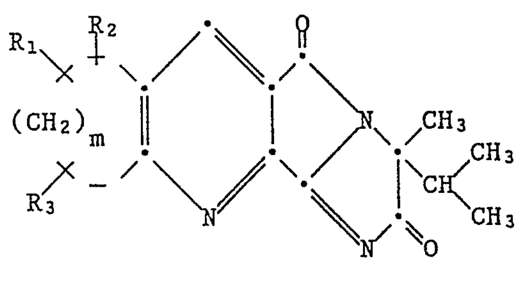

(Ic)

worin $R_1$, $R_2$, $R_3$ und m wie zuvor definiert ist.

Als Einzelverbindungen zu nennen sind:
7H-5,6-Dihydro-2-(5-isopropyl-5-methyl-1H-imidazol-4-on-2-yl)-5-methyl-1-pyrindin-3-carbonsäure,
7H-5,6-Dihydro-2-(5-isopropyl-5-methyl-1H-imidazol-4-on-2-yl)-6-methyl-1-pyrindin-3-carbonsäure,
2-(5-Isopropyl-5-methyl-1H-imidazol-4-on-2-yl)-5-methyl-5,6,7,8-tetrahydrochinolin-3-carbonsäure,
2-(5-Isopropyl-5-methyl-1H-imidazol-4-on-2-yl)-7-methyl-5,6,7,8-tetrahydrochinolin-3-carbonsäure,
2-(5-Isopropyl-5-methyl-1H-imidazol-4-on-2-yl)-8-methyl-5,6,7,8-tetrahydrochinolin-3-carbonsäure,
2-(5-Isopropyl-5-methyl-imidazol-4-on-2-yl)-5,6,7,8-tetrahydro-5,7,7-trimethylchinolin-3-carbonsäure,
2-(5-Isopropyl-5-methyl-1H-imidazol-4-on-2-yl)-6-methyl-5,6,7,8-tetrahydrochinolin-3-carbonsäure,
2,8-Dimethyl-2-isopropyl-7,8,9,10-tetrahydro-3H,5H-imidazo[1′,2′:1,2]-pyrrolo[3,4-b]chinolin-3,5-dion,
2,7-Dimethyl-2-isopropyl-7,8,9,10-tetrahydro-3H,5H-imidazo[1′,2′:1,2]-pyrrolo[3,4-b]chinolin-3,5-dion,

2,9-Dimethyl-2-isopropyl-7,8,9,10-tetrahydro-3H,5H-imidazo[1′,2′:1,2]-pyrrolo[3,4-b]chinolin-3,5-dion,
6,6-Dimethyl-2-(5-isopropyl-5-methyl-1H-imidazol-4-on-2-yl)-5,6,7,8-tetrahydrochinolin-3-carbonsäure,
6,6-Dimethyl-2-(5-isopropyl-5-methyl-1H-imidazol-4-on-2-yl)-3-methoxycarbonyl-5,6,7,8-tetrahydrochinolin,
2-(5-Isopropyl-5-methyl-1H-imidazol-4-on-2-yl)-3-methoxycarbonyl-7-methyl-5,6,7,8-tetrahydrochinolin und,
2-Isopropyl-7,8,9,10-tetrahydro-2,8,8-trimethyl-3H,5H-imidazo[1′,2′:1,2]-pyrrolo[3,4-b]chinolin-3,5-dion.

Besonders bevorzugt sind auch die Verbindungen der Formel Ia, in denen A für OH steht, sowie deren Salze

Die anmeldungsgemässen Verbindungen der Formel I können analog zu literaturbekannten Verfahren hergestellt werden. In Abhängigkeit von der Struktur des jeweils gewünschten Endprodukts der Formel Ia, Ib oder Ic können verschiedene an sich bekannte Verfahren angewendet werden.

Die Erfindung betrifft somit auch Verfahren zur Herstellung von Verbindungen der Formel Ia

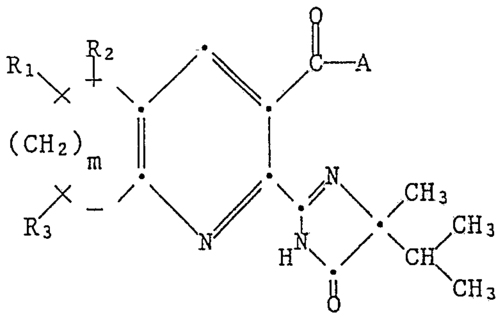

(Ia)

worin A für OH steht und die Reste X, $R_1$, $R_2$, $R_3$ und m wie zuvor definiert sind,
dadurch gekennzeichnet, dass man

a) einen Diester der Formel II

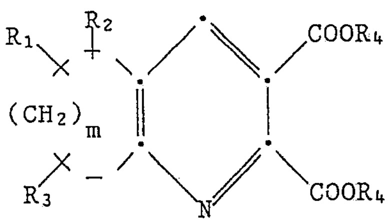

(II)

worin $R_1$, $R_2$, $R_3$ und m wie zuvor definiert sind und die Reste $R_4$ unabhängig voneinander für $C_1$-$C_6$-Alkyl stehen mit einem 2-Amino-2,3-dimethylbuttersäureamid der Formel III

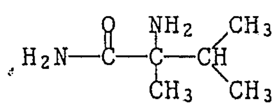

(III)

in Gegenwart einer Base umsetzt.

Des weiteren betrifft die Erfindung Verfahren zur Herstellung von Verbindungen der Formel Ib

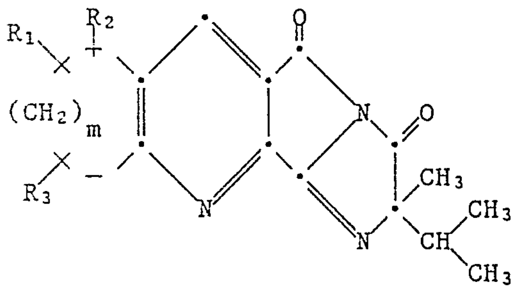

(Ib)

worin $R_1$, $R_2$, $R_3$ und m wie zuvor definiert sind, dadurch gekennzeichnet, dass man

b) eine Verbindung der Formel Ia

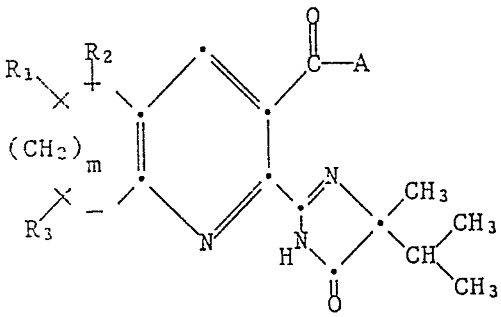

(Ia)

worin $R_1$, $R_2$, $R_3$ und m wie zuvor definiert sind und A für OH steht in Gegenwart eines wasserbindenden bzw. wasserabscheidenden Mittels zu Ib cyclisiert.

Ausserdem betrifft die Erfindung Verfahren zur Herstellung von Verbindungen der Formel Ic

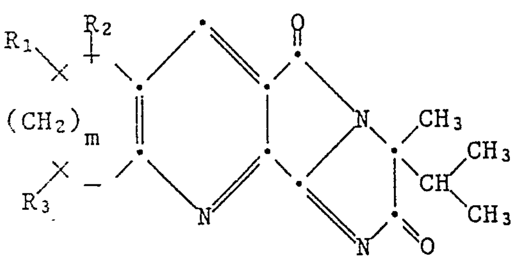

(Ic)

worin $R_1$, $R_2$, $R_3$ und m wie zuvor definiert sind, dadurch gekennzeichnet, dass man

c) ein Säureanhydrid der Formel IV

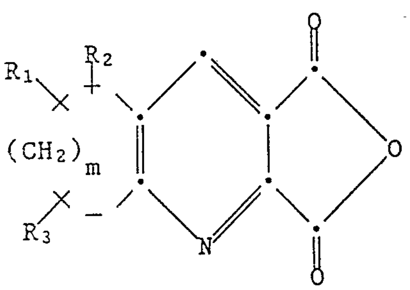

(IV)

worin $R_1$, $R_2$, $R_3$ und m wie zuvor definiert sind, mit 2-Amino-2,3-dimethylbuttersäureamid der Formel III

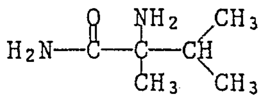

(III)

zu einem Imid der Formel V

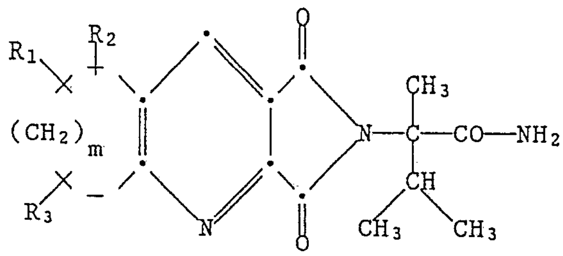

(V)

umsetzt und das so erhältliche Imid der Formel V in Gegenwart einer Base unter Wasserabspaltung zu Ic cyclisiert oder

d) eine Verbindung der Formel Ib

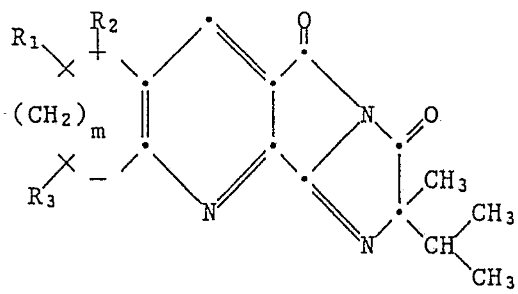

(Ib)

worin $R_1$, $R_2$, $R_3$ und m wie zuvor definiert sind zu Ic isomerisiert.

Weiterhin betrifft die Erfindung ein Verfahren zur Herstellung von Verbindungen der Formel Ia

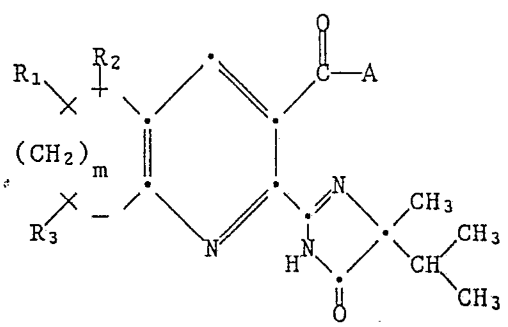

(Ia)

worin $R_1$, $R_2$, $R_3$ und m wie zuvor definiert sind und A für $C_1$-$C_4$-Alkoxy steht, dadurch gekennzeichnet, dass man

e) eine Verbindung der Formel Ib

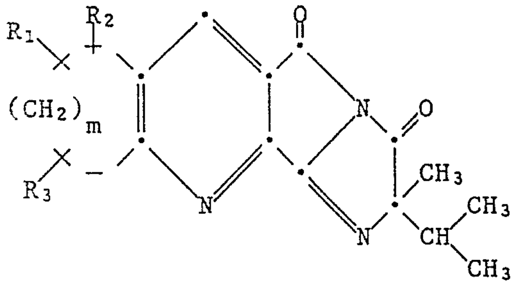

(Ib)

oder eine Verbindung der Formel Ic

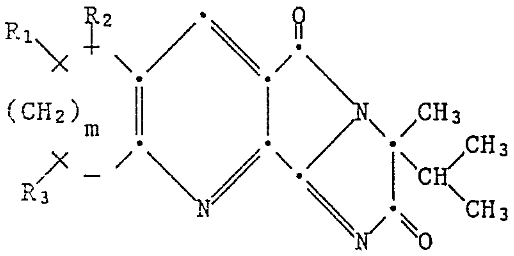

(Ic)

in welchen die Reste $R_1$, $R_2$, $R_3$ und m wie zuvor definiert sind, mit einem Alkohol der Formel VI
H A VI,
worin A für $C_1$-$C_4$-Alkoxy steht, gewünschtenfalls in Gegenwart einer Säure oder Base umsetzt, oder
f) einen Diester der Formel II

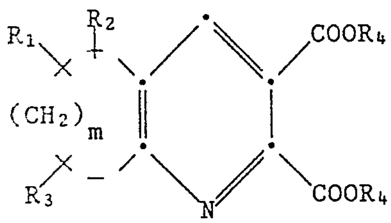

(II)

in dem $R_1$, $R_2$, $R_3$ und m wie zuvor definiert sind und $R_4$ für $C_1$-$C_4$-Alkyl steht, unter nicht hydrolisierenden Bedingungen und in Gegenwart einer Base mit 2-Amino-2,3-dimethylbuttersäureamid umsetzt.
Die Erfindung umfasst auch Verfahren zur Herstellung von Salzen der Formel Ia'

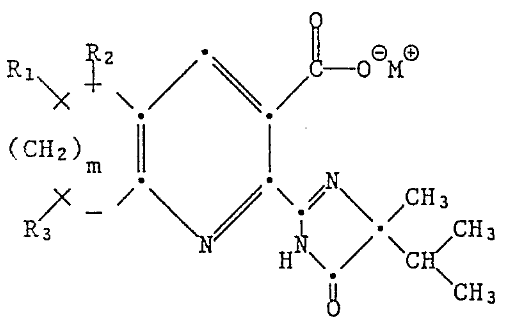

(Ia')

worin $R_1$, $R_2$, $R_3$ und m wie zuvor definiert sind und $M^{\oplus}$ für ein Kationenäquivalent eines Alkali- oder Erdalkalimetalls oder einer stickstoffhaltigen Base steht,

dadurch gekennzeichnet, dass man

g) eine Verbindung der Formel Ia

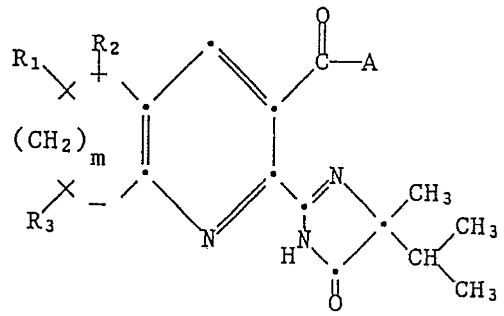

(Ia)

in der die Reste $R_1$, $R_2$, $R_3$ und m wie zuvor definiert sind und A für OH oder $C_1$-$C_4$-Alkoxy steht mit einer äquivalenten Menge einer Verbindung der Formel VII

$OH^{\ominus}M^{\oplus}$ (VII)

in der $M^{\oplus}$ für ein Kationenäquivalent eines Alkali- oder Erdalkalimetalls oder eine quaternäre stickstoffhaltige Base steht, umsetzt, oder

h) eine Verbindung der Formel Ia, in der A für OH steht mit einem Alkali-oder Erdalkalimetall, einem Alkali oder Erdalkalisalz oder der stickstoffhaltigen Base direkt umsetzt.

Zu den erfindungsgemässen Verfahren analoge Umsetzungen, sowie die bei deren Durchführung zu beachtenden Reaktionsbedingungen, wie Auswahl von Lösungsmitteln und Reaktanden, sind beispielsweise aus den folgenden Patentpublikationen bekannt geworden: EP-A-0041623, EP-A-0212200, EP-A-95105, GB-A-2174395, DE-A-3420271, EP-A-0133309, EP-A-0216360, DE-A-3616894, US 4701208.

Die Umsetzungen erfolgen im allgemeinen zwischen 0 und 180°C, vorzugsweise im Temperaturbereich zwischen Raumtemperatur und der Rückflusstemperatur der Reaktionsgemische.

Bevorzugt werden die Reaktionen in Gegenwart von Lösungsmitteln durchgeführt. Geeignete Lösungsmittel sind insbesondere diejenigen, welche die Edukte und/oder Produkte zu lösen vermögen und welche die Reaktion nicht nachteilig beeinflussen.

Zu nennen sind Alkohole, wie Methanol, Ethanol, n-Propanol, Iso-Propanol, n-Butanol, tert.-Butanol, Glyme, Ethylenglycol etc.; Ether, wie Diethylether, Tetrahydrofuran, Diglyme etc; Halogenkohlenwasserstoffe, wie Dichlormethan, Trichlormethan, Tetrachlormethan etc; aromatische Kohlenwasserstoffe, wie Benzol, Toluol, Xylol, Chlorbenzol, Dichlorbenzol etc; Säureamide, wie Dimethylformamid, Acetamid, N-Methylpyrrolidon; Sulfoxide, wie Dimethylsulfoxid oder Sulfolan; oder andere gegenüber den Reaktionspartnern inerte Lösungsmittel.

In einigen der zuvor genannten Reaktionen ist es vorteilhaft wasserfrei zu arbeiten (Verfahren b, c, d, oder e, während in Verfahren h Wasser allein als Lösungsmittel oder Wasser-Lösungsmittelgemische zur Anwendung kommen können.

Geeignete Basen sind u.a. Alkoholate, Amine, Hydride, Amide oder metallorganische Verbindungen.

Protonensäuren sind Halogenwasserstoffsäuren, Schwefelsäure, Phosphorsäure etc.

Als wasserbindendes Mittel hat sich Dicyclohexylcarbodiimid (DCC), insbesondere das System DCC/Dichlormethan, bewährt.

Als wasserabscheidende Mittel werden unter anderem die in der präparativen Chemie üblichen Verfahrensmassnahmen des Auskreisens von Wasser mittels azeotroper Gemische, wie etwa Toluol/Wasser, vorgeschlagen.

Die Ausgangsverbindungen der Formel II, IV und V sind ebenfalls neu und bilden einen weiteren Gegenstand der Erfindung.

Verbindung II ist herstellbar:

a) durch Umsetzung eines Enamins der Formel VIII

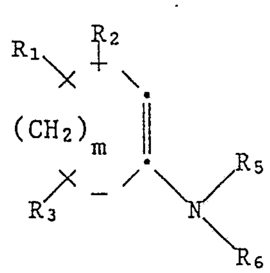

(VIII)

worin $R_5$ und $R_6$ unabhängig voneinander für $C_1$-$C_4$-Alkyl oder $R_5$ und $R_6$ gemeinsam eine, gewünschtenfalls einmal durch Sauerstoff oder N-$R_7$ (mit $R_7$ = Wasserstoff oder $C_1$-$C_4$-Alkyl) unterbrochene $-(CH_2)_p-$Kette bedeutet mit p = 4-6 und $R_1$, $R_2$, $R_3$ und m wie zuvor definiert sind, mit einem Diester der Formel IX

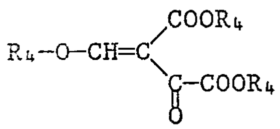

(IX)

worin $R_4$ für $C_2$-$C_4$-Alkyl steht, und das aus VIII und IX erhältliche Monokondensationsprodukt in einem zweiten Reaktionsschritt mit Ammoniak zu II cycliert wird oder

b) durch Kondensation eines β-Hydroxyketons der Formel X

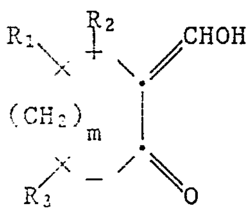

(X)

worin $R_1$, $R_2$, $R_3$ und m wie zuvor definiert sind mit einem Aminofumarat XI

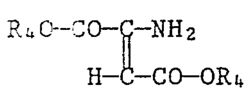

(XI)

worin $R_4$ wie zuvor definiert ist.

Die Enamine der Formel VIII sind bekannt oder können analog zu literaturbekannten Verfahren durch sauer katalysierte Kondensation eines sekundären Amins mit dem jeweiligen Keton hergestellt werden.

Im Falle unsymmetrisch substituierter Ketone entstehen so Enamingemische, die bei der Kondensationsreaktion a) zu Gemischen isomerer Diester II führen.

Die Auftrennung dieser konstitutionsisomeren Verbindungen II kann sowohl auf der Stufe des Diesters, wie auch auf der Stufe der aus dem Diester herstellbaren Produkte I, IV bzw. V erfolgen.

Die Verfahrensvariante b) ist unter anderem aus GB-A-2174395 bekannt.

Die Anhydride der Formel IV sind analog zu den vorstehend genannten Patentpublikationen herstellbar

a) durch Verseifung eines Esters der Formel II

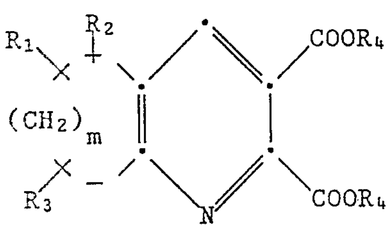

(II),

worin $R_1$, $R_2$, $R_3$, $R_4$ und m wie zuvor definiert sind, zu einer Dicarbonsäure der Formel XII

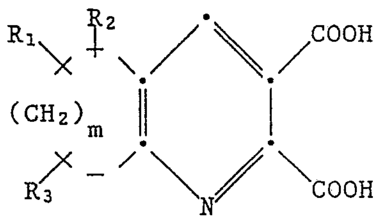

(XII),

worin $R_1$, $R_2$, $R_3$ und m wie zuvor definiert sind, und anschliessender Cyclisierung dieser Dicarbonsäure XII mit wasserabspaltenden Mitteln zu IV.

Die Imide der Formel V sind erhältlich

a) durch Umsetzung eines Anhydrides der Formel IV

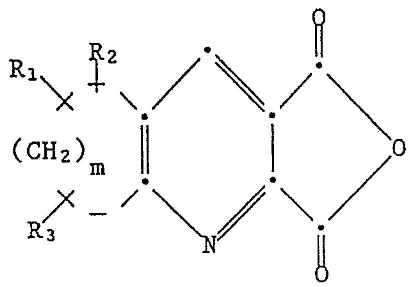

(IV),

worin $R_1$, $R_2$, $R_3$ und m wie zuvor definiert sind, mit 2-Amino-2,3-dimethylbuttersäureamid III

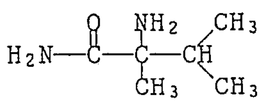

(III).

Die neuen Dicarbonsäuren der Formel X sowie deren Alkali- und Erdalkalisalze sind ebenfalls Gegenstand der Erfindung. Die zur Durchführung vorstehender Verfahren notwendigen Lösungsmittel und Reaktionsbedingungen sind dem Fachmann geläufig und entsprechen im Wesentlichen den im Zusammmenhang mit der Herstellung der Endprodukte Genannten.

Ein weiterer Gegenstand der Erfindung sind herbizide Mittel enthaltend mindestens eine Verbindung der Formel I als Aktivsubstanz, die Verwendung von Verbindungen der Formel I in Verfahren zur pre-und/oder postemergenten Bekämpfung von Unkräutern und Gräsern sowie die Herstellung herbizider Mittel enthaltend Verbindungen der Formel I.

Als Herbizide werden die Wirkstoffe der Formel I in der Regel mit Aufwandmengen von 0,005 bis 5, insbesondere 0,05 bis 4,0 kg/ha, insbesondere 0,1 bis 2,0 kg/ha, erfolgreich eingesetzt. Die Herbizidwirkung ist abhängig von der Aufwandmenge. Bei hohen Aufwandmengen zeigen die Verbindungen der Formel I totalherbizide, bei verringerten Aufwandmengen selektivherbizide Wirkung. Die für den jeweiligen Verwendungszweck und Anwendungszeitpunkt optimale Wirkstoffmenge bei preemergenter bzw. postemergenter Anwendung kann durch Versuche ermittelt werden. Des weiteren können die Verbindungen der Formel I auch auf das Saatgut der jeweiligen Kulturpflanze aufgebracht werden (Saatgutbeizung).

Die Verbindungen der Formel I zeichnen sich durch Selektivität bei Leguminosen und Soja aus. Die Verbindungen der Formel I werden daher vorgeschlagen zum Einsatz in dicotylen Nutzpflanzenkulturen, insbesondere pre- und postemergent in Soja und postemergent in Baumwolle.

Neben der herbiziden Wirkung verschiedenste Unkräuter und Gräser sind die Verbindungen der Formel I auch pflanzenwuchsregulatorisch wirksam.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Strassenrändern, auf Flughäfen oder in Obstanlagen reduziert werden. Von Bedeutung ist auch die Hemmung des Wuchses von krautigen und holzigen Pflanzen an Strassenrändern und in der Nähe von Pipelines oder Ueberlandleitungen oder ganz allgemein in Bereichen, in denen ein starker Zuwachs der

Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne dass die Gefar besteht, dass das Getreide lagert.

Eine Hemmung des vegetativen Wachstums ermöglicht bei vielen Kulturpflanzen eine dichtere Anpflanzung, so dass Mehrerträge bezogen auf die Bodenfläche erzielt werden können. Ein Vorteil der so erzielten kleineren Pflanzen ist auch, dass die Kultur leichter bearbeitet und beerntet werden kann.

Die Verbindungen der Formel I sind insbesondere bei Monocotyledonen wuchsregulatorisch wirksam.

In besonders vorteilhafter Weise können Verbindungen der Formel I zur Wachstumsregulation von Untersaaten in Maiskulturen verwendet werden.

Als Untersaaten in Maiskulturen sind prinzipiell diejenigen Pflanzen geeignet, die auf dem Boden zwischen den einzelnen Maispflanzen wachsen und so in erster Linie der Bodenerosion in Maiskulturen entgegenwirken.

Geeignete Pflanzen für die Untersaat sind unter anderem Raps. Klee, Gräser und Leguminosen.

Verbindungen der Formel I zeigen in vorteilhafter Weise eine "pre-post-Selektivität" bei verscheidenen Gräsern in Maiskulturen.

Die wuchshemmende Wirkung in Gräsern ist in postemergenter Applikation stark ausgeprägt, während bei gleichzeitiger Preemergenter Applikation Maispflanzen in ihrem Wuchs nur geringfügig gehemmt werden. Derartige "pre-post-Selektivitäten" erlauben somit die Aussaat von Mais in Gräsern. Dabei soll der Zeitpunkt für die Applikation der Aktivsubstanz der Formel I so gewählt werden, dass die Aktivsubstanz postemergent für die Gräser ober preemergent für den Mais angewendet wird. In den ersten Wochen ist der Zuwachs der Gräser so stark gehemmt, dass die Maispflanze sich ungehindert entfalten kann.

Als Verbindungen der Formel I mit ausgeprägter "pre-post-Selektivität" bei Untersaaten in Mais sind 2-(5-Isopropyl-5-methyl-1H-imidazol-4-on-2-yl)-5,5-dimethyl-5,6,7,8-tetrahydrochinolin-3-carbonsäure, 2,7-Dimethyl-2-isopropyl-7,8,9,10-tetrahydro-3H,5H-imidazo[1′,2′:1,2]pyrrolo[3,4-b]chinolin-3,5-dion, 2,9-Dimethyl-2-isopropyl-7,8,9,10-tetrahydro-3H,5H-imidazo[1′,2′:1,2]pyrrolo[3,4-b]chinolin-3,5-dion und das Gemisch aus 2,7(9)-Dimethyl-2-isopropyl-7,8,9,10-tetrahydro-3H,5H-imidazo[1′,2′:1,2]pyrrolo[3,4-b]chinolin-3,5-dion hervorzuheben.

Die Verbindungen der Formel I werden insbesondere bei Aufwandmengen um 0,005 bis 1 kg/ha erfolgreich als Wuchsregulatoren eingesetzt. Die für den jeweiligen Verwendungszweck und Anwendungszeitpunk optimale Wirkstoffmengen bei preemergenter bzw. postemergenter Anwendung kann durch Versuche ermittelt werden.

Ausserdem zeigen die Verbindungen der Formel I bei postemergenter Applikation bei einzelnen Kulturpflanzen wie Baumwolle, Gerste, Weizen, Raps oder Sonnenblume sowie die monokotyledonen Gräsern, wie Poa, Festuca, Lolium, Bromus und Cynosurus, gute wuchsregulatorische Effekte bei gleichzeitig geringer nekrotischer Wirkung. Damit können die Verbin dungen der Formel I in vorteilhafter Weise als postemergente Wuchsregulatoren in Getreide und Gräsern sowie Sonnenblumen und Raps eingesetzt werden.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I können auch auf das Saatgut der Nutzpflanzenkultur aufgetragen werden (Samenbeizung). Die herbizide oder wuchsregulatorische Aktivsubstanz wird dann mit der Aussaat der Nutzpflanze auf das Feld gebracht. Das mit einer wirksamen Menge an Aktivsubstanz der Formel I behandelte Saatgut ist ein weiterer Gegenstand der Erfindung.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclo hexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige

Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier-und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfonate liegen in der Regel als Alkali-, Erdalkali-oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykoletherund -esterderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethanol Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl-oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:
"1986 International Mc Cutcheon's Emulsifiers & Detergents" Glen Rock, NJ, USA, 1986.
Dr. Helmut Stache "Tensid Taschenbuch"
Carl Hanser Verlag, München/Wien 1981.

Die Formulierungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

Emulgierbare Konzentrate:

| | |
|---|---|
| Wirkstoff der Formel I: | 1 bis 20 % bevorzugt 5 bis 10 % |
| oberflächenaktives Mittel: | 5 bis 30 %,vorzugsweise 10 bis 20 % |
| flüssiges Trägermittel: | 50 bis 94 %,vorzugsweise 70 bis 85 %. |

Stäube:

| | |
|---|---|
| Wirkstoff der Formel I: | 0,1 bis 10%, vorzugsweise 0,1 bis 1 % |
| festes Trägermittel: | 99,9 bis 90 %, vorzugsweise 99,9 bis 99 %. |

Suspensions-Konzentrate:

| | |
|---|---|
| Wirkstoff der Formel I: | 5 bis 75 %, vorzugsweise 10 bis 50 % |
| Wasser: | 94 bis 25 %, vorzugsweise 90 bis 30 % |
| oberflächenaktives Mittel: | 1 bis 40 %, vorzugsweise 2 bis 30 %. |

Benetzbare Pulver:

| | |
|---|---|
| Wirkstoff der Formel I: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 15 bis 90 %. |

Granulate:

| | |
|---|---|
| Wirkstoff der Formel I: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg Aktivsubstanz pro ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachstehenden Beispiele erläutern den Erfindungsgegenstand.

## H. Herstellungsbeispiele

### H.1. Verbindungen der Formel II

#### H.1.1. 6-Methyl-5,6,7,8-tetrahydrochinolin-2,3-dicarbonsäurediethylester

56 g 4-Methylcyclohexanon werden in Cyclohexan zusammen mit 53,5 g Morpholin und 0,2 g p-Toluolsulfonsäure an einem Wassabscheider bis zur Beendigung der Wasserabscheidung zum Sieden erhitzt.

Nach Abdestillierenn des Lösungsmittels wird im Vakuum destilliert. Man isoliert 75 g 4-(4-Methylcyclohexen-1-yl)-morpholin (Kp. 73°C/0,08 mm Hg).

27,2 g des so erhältlichen Enamins werden in 50 ml Ethanol mit 37,0 g Ethoxymethylenoxalylessigsäurediethylester versetzt und für mehrere Stunden bei Raumtemperatur belassen. Danach werden 2,8 g Ammoniak (als gesättigte alkoholische Lösung) zugegeben und für 10 min zum Sieden erhitzt.
Nach dem Erkalten und dem Abdestillieren des Lösungsmittels im Vakuum wird der Rückstand an Kieselgel mit Essigsäureethylester/ Hexan (1:2) chromatographiert.

Man isoliert 25,5 g der Titelverbindung der Formel

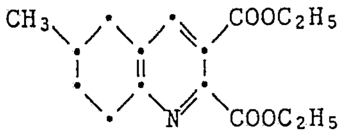

als gelbliches Oel (Verbindung 1.01).

H.1.2. 7H-5,6-Dihydro-5(6)-methyl-1-pyrindin-2,3-dicarbonsäurediethylester

39 g rac. 3-Methylcyclopentanon werden mit 41 g Morpholin und einer Spatelspitze p-Toluolsulfonsäure in Cyclohexan an einem Wasserabscheider bis zur Beendigung der Wasserabscheidung zum Sieden erhitzt. Nach dem Abdestillieren des Lösungsmittels wird im Vakuum fraktioniert.

Man isoliert 50,5 g einer Mischung aus 4-(3-Methyl-cyclopenten-1-yl)-morpholin und 4-(4-Methyl-cyclopenten-1-yl)-morpholin.

20 g der so erhältlichen Enaminmischung werden in 30 ml Ethanol mit 14,65 g Ethoxymethylenoxalylessigsäurediethylester versetzt und für 2 Stunden bei Raumtemperatur belassen. Danach wird eine äquivalente Menge Ammoniak (als gesättigte ethanolische Lösung) zugegeben und für einige Zeit bei Raumtemperatur belassen.

Danach wird das Lösungsmittelgemisch abdestilliert, der Rückstand auf Eiswasser gegossen und in Essigsäureethylester aufgenommen. Die organische Phase wird mit verdünnter Essigsäure gewaschen, eingeengt und im Kugelrohr destilliert.

Man isoliert 10,5 g der Titelverbindung als Gemisch der Verbindungen der Formel

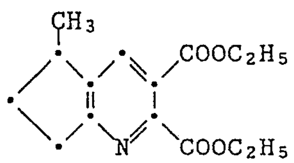

und

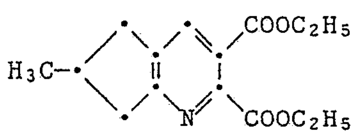

als Oel mit Sdp. 220°C (0,1 mm Hg) (Verbindung 1.06 und 1.07).

H.1.3. 5(7)-Methyl-5,6,7,8-tetrahydrochinolin-2,3-dicarbonsäure-diethylester

Analog zu Beispiel H.1.2. erhält man aus 56 g (3-Methylcyclohexanon und 53,5 g Morpholin 73 g eines Gemisches aus 4-(3-Methylcyclohexen-1-yl)-morpholin und 4-(5-Methylcyclohexen-1-yl)-morpholin.

Aus 27,2 g dieses Enamingemisches erhält man analog zu Beispiel H.1.2. 30,5 g der Titelverbindung als Gemisch der Verbindungen der Formel

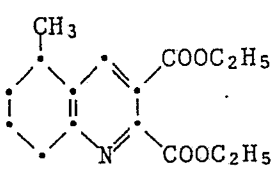

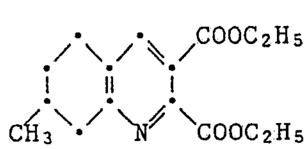

(Hauptprodukt)

als gelbes Oel (Verbindung 1.02 und 1.03).

H.1.4. 8-Methyl-5,6,7,8-tetrahydrochinolin-2,3-dicarbonsäurediethylester

100 ml 2-Methylcyclohexanon, 87 g Morpholin und 0,5 g p-Toluolsulfonsäure werden in Cyclohexan an einem Wasserabscheider bis zur Beendigung der Wasserabscheidung zum Sieden erhitzt.

Danach wird das Reaktionsgemisch destillativ aufgearbeitet. Bei einem Kp. von 99-108° C (14 mm Hg) isoliert man eine Mischung aus 4-(2-Methylcyclohexen-1-yl)-morpholin und 4-(6-Methylcyclohexen-1-yl)-morpholin.

18,1 g diese Enamingemisches werden in 100 ml Ethanol gelöst und mit 24,4 g Ethoxymethylenoxalylessigsäurediethylester versetzt und 1 Stunde zum Sieden erhitzt. Danach wird eine äquivalente Menge Ammoniak (als gesättigte alkoholische Lösung) zugegeben und noch weitere 30 min zum Sieden erhitzt. Nach Abdestillieren des Lösungsmittels wird das Produkt an Kieselgel mit Essigester/Hexan (1:4) chromatographiert.

Man isoliert 6,5 g der Titelverbindung der Formel

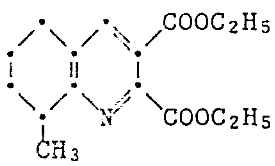

als helles Oel (Verbindung 1.04).

H.1.5. 5,6,7,8-Tetrahydro-5,7,7-trimethylchinolin-2,3-dicarbonsäurediethylester

Analog Beispiel H.1.4 werden aus 100 g 3,3,5-Trimethylcyclohexanon und 63 g Morpholin 82 g eines Enamins mit Kp. 120-121° C (15 mm Hg) erhalten.

Aus 16,0 g des so hergestellten Enamins sind analog zu Beispiel H.1.4. 76 g der Titelverbindung der Formel

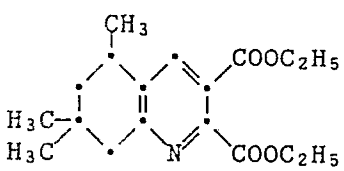

erhältlich (Verbindung 1.05).

Analog zu den vorstehenden Beispielen H.1.1. bis 1.5. sind die Verbindungen der Tabelle I herstellbar:

Verbindungen der Formel II

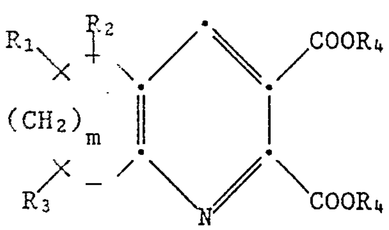

(II).

Tabelle I

| Verb.No. | m | $R_1$ | $R_2$ | $R_3$ | $R_4$ | phys. Daten |
|---|---|---|---|---|---|---|
| 1.01 | 4 | 6-$CH_3$ | H | H | $C_2H_5$ | Oel |
| 1.02 | 4 | 5-$CH_3$ | H | H | $C_2H_5$ | Oel[2] |
| 1.03 | 4 | 7-$CH_3$ | H | H | $C_2H_5$ | Oel[2] |
| 1.04 | 4 | 8-$CH_3$ | H | H | $C_2H_5$ | Oel |
| 1.05 | 4 | 5-$CH_3$ | 7-$CH_3$ | 7-$CH_3$ | $C_2H_5$ | |
| 1.06 | 3 | 5-$CH_3$ | H | H | $C_2H_5$ | Kp.$_{(0,1)}$:220°C[1] |
| 1.07 | 3 | 6-$CH_3$ | H | H | $C_2H_5$ | Kp.$_{(0,1)}$:220°C[1] |
| 1.08 | 4 | 8-$CH_3$ | 8-$CH_3$ | H | $C_2H_5$ | |
| 1.09 | 4 | 6-$CH_3$ | 8-$CH_3$ | H | $C_2H_5$ | |
| 1.10 | 4 | 5-$CH_3$ | 7-$CH_3$ | H | $C_2H_5$ | |
| 1.11 | 4 | 7-$CH_3$ | 7-$CH_3$ | H | $C_2H_5$ | |
| 1.12 | 4 | 5-$CH_3$ | 5-$CH_3$ | H | $C_2H_5$ | |
| 1.13 | 4 | 5-$CH_3$ | 6-$CH_3$ | 8-$CH_3$ | $C_2H_5$ | |
| 1.14 | 4 | 8-$(CH_2)_2$-8 | | H | $C_2H_5$ | |
| 1.15 | 3 | 7-$(CH_2)_2$-7 | | H | $C_2H_5$ | |
| 1.16 | 4 | 8-$(CH_2)_2$-8 | | 6-$CH_3$ | $C_2H_5$ | |
| 1.17 | 3 | 7-$CH_3$ | H | H | $C_2H_5$ | |
| 1.18 | 3 | 7-$CH_3$ | 7-$CH_3$ | H | $C_2H_5$ | |
| 1.19 | 3 | 5-$CH_3$ | 7-$CH_3$ | H | $C_2H_5$ | |
| 1.20 | 3 | 6-$CH_3$ | 7-$CH_3$ | H | $C_2H_5$ | |
| 1.21 | 3 | 6-$CH_3$ | 6-$CH_3$ | H | $C_2H_5$ | |
| 1.22 | 3 | 6-$CH_3$ | H | H | $C_2H_5$ | |

[1]Verbindung 1.06 und 1.07 als Gemisch isoliert.
[2]Verbindung 1.02 und 1.03 als Gemisch isoliert.

H.2. Verbindungen der Formel Ia

H.2.1. 2-(5-Isopropyl-5-methyl-1H-imidazol-4-on-2-yl)-6-methyl-5,6,7,8-tetrahydrochinolin-3-carbonsäure

23,0 g 6-Methyl-5,6,7,8-tetrahydrochinolin-2,3-dicarbonsäurediethylester werden zusammen mit 12,0 g 2-Amino-2,3-dimethylbuttersäureamid und 20 g Kalium-tert.-butanolat in 200 ml Toluol 5 Stunden bei 80°C gerührt. Nach dem Erkalten wird das Reaktionsgemisch mehrmals mit Wasser extrahiert. Die vereinigten Wasserextrakte werden mit 20 ml Eisessig versetzt und ihrerseits mit Dichlormethan mehrfach extrahiert. Nach Abdestillieren des Dichlormethans wird der Rückstand mit Diethylether digeriert.

Man isoliert 17,0 g der Titelverbindung der Formel

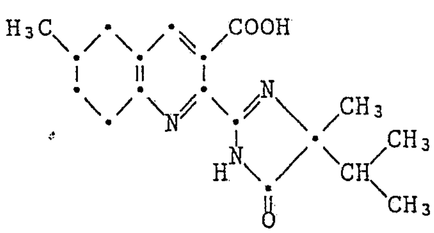

als Kristalle vom Smp. 152-158°C, (Verbindung 2.01).

H.2.2. 7H-5,6-Dihydro-2-(5-isopropyl-5-methyl-1H-imidazol-4-on-2-yl)-5(6)-methyl-1-pyrindin-3-carbonsäure

10,4 g des nach H.1.2. erhältlichen Diestergemisches werden zusammen mit 5,2 g 2-Amino-2,3-dimethylbuttersäureamid und 9 g Kalium-tert.-butylat in 150 ml Toluol für 3 Stunden zum Sieden erhitzt. Nach dem Erkalten wird mehrmals mit 50 ml Wasser extrahiert. Die Wasserextrakte werden mit Salzsäure auf pH 3 gestellt und mehrfach mit Dichlormethan extrahiert. Nach Abdestillieren des Lösungsmittels wird der Rückstand aus Diethylether umkristallisiert.

Man isoliert 6,0 g der Titelverbindung als Gemisch der Verbindungen der Formel

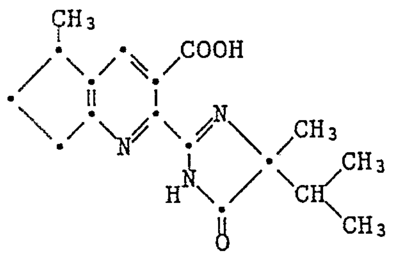

und

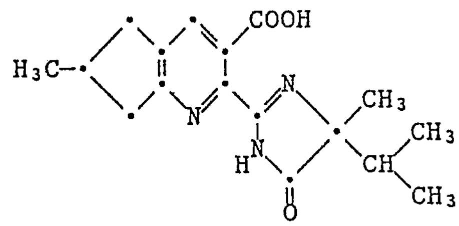

,

als Kristalle von Smp. 75-85°C (Verbindungen 2.06 und 2.07).

H.2.3. 2-(5-Isopropyl-5-methyl-1H-imidazol-4-on-2-yl)-5(7)-methyl-5,6,7,8-tetrahydrochinolin-3-carbonsäure

Ausgehend von 27,0 g des nach Beispiel H.1.3. erhältlichen Diesters und 14 g 2-Amino-2,3-dimethylbuttersäureamid erhält man analog zu Beispiel H.2.2. 16,0 g der Titelverbindung als Gemisch der Verbindungen der Formel

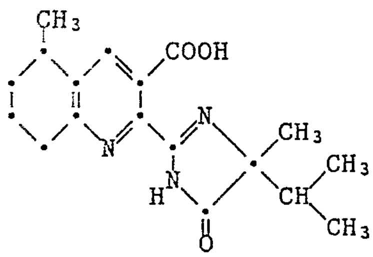

und

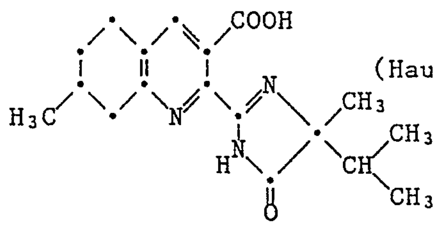

(Hauptprodukt)

mit dem Smp. 159-163°C (Verbindung 2.02 und 2.03).

H.2.4. 2-(5-Isopropyl-5-methyl-1H-imidazol-4-on-2-yl)-8-methyl-5,6,7,8-tetrahydrochinolin-3-carbonsäure

4,3 g des nach Beispiel H.1.4. erhältlichen Diesters werden in 200 ml Toluol gelöst und mit 2,0 g 2-Amino-2,3-dimethylbuttersäureamid in Gegenwart von 3,5 g Kalium-tert.-butylat für 2 Stunden auf 100°C erhitzt. Es wird analog Beispiel H.2.2. aufgearbeitet.

Man isoliert 2,8 g der Titelverbindung der Formel

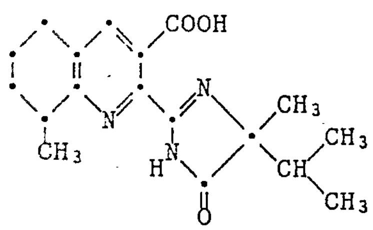

als Kristalle von Smp. 189-196°C (Verbindung 2.04).

H.2.5. 2-(5-Isopropyl-5-methyl-imidazol-4-on-2-yl)-5,6,7,8-tetrahydro-5,7,7-trimethyl-chinolin-3-carbonsäure

Aus 7,0 g des Diesters gemäss Beispiel H.1.5. erhält man analog zu Beispiel H.2.2. 3,8 g der Titelverbindung der Formel

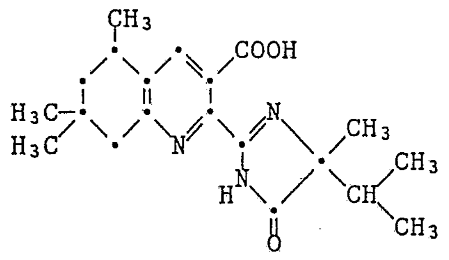

als Kristalle vom Smp. 170-180°C (Verbindung 2.05).

H.2.6. 2-(5-Isopropyl-5-methyl-imidazol-4-on-2-yl)-3-methoxycarbonyl-7-methyl-5,6,7,8-tetrahydrochinolin

12,0 g des nach Herstellungsbeispiel H.3.2. erhältlichen Gemisches werden in 100 ml Methanol gelöst, mit 2,0 g einer 30%-igen Natriummethanolatlösung (in Methanol) versetzt und 3 1/2 Stunden zum Sieden erhitzt. Danach wird mit 0,45 ml Eisessig versetzt zur Trockene eingedampft und der Rückstand in Diethylether aufgenommen. Die etherische Lösung wird erneut eingedampft und mit Petrolether digeriert.

Man isoliert 8,8 g der Titelverbindung der Formel

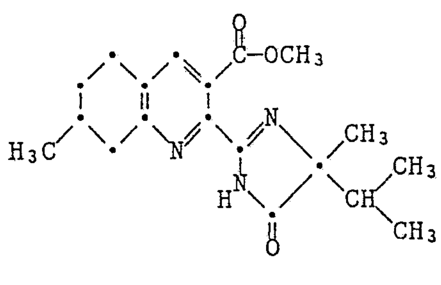

als Kristalle vom Smp. 138-140°C (Verb. No. 2.26).

Analog zu den vorstehenden Beispielen H.2.1. bis H.2.5. sind die Verbindungen der Tabelle II herstellbar.

Verbindungen der Formel Ia

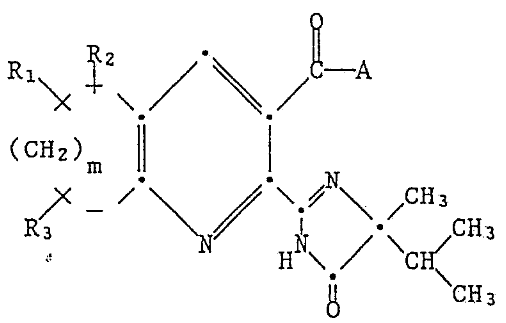

(Ia).

Tabelle II

| Verb.No. | m | $R_1$ | $R_2$ | $R_3$ | A | phys. Daten |
|---|---|---|---|---|---|---|
| 2.01 | 4 | 6-$CH_3$ | H | H | OH | Fp. 152-158°C |
| 2.02 | 4 | 5-$CH_3$ | H | H | OH | Fp. 159-163°C[2)] |
| 2.03 | 4 | 7-$CH_3$ | H | H | OH | Fp. 159-163°C[2)] |
| 2.04 | 4 | 8-$CH_3$ | H | H | OH | Fp. 189-196°C |
| 2.05 | 4 | 5-$CH_3$ | 7-$CH_3$ | 7-$CH_3$ | OH | Fp. 170-180°C |
| 2.06 | 3 | 5-$CH_3$ | H | H | OH | Fp. 75-85°C[1)] |
| 2.07 | 3 | 6-$CH_3$ | H | H | OH | Fp. 75-85°C[1)] |
| 2.08 | 4 | 8-$CH_3$ | 8-$CH_3$ | H | OH | |
| 2.09 | 4 | 6-$CH_3$ | 8-$CH_3$ | H | OH | |
| 2.10 | 4 | 5-$CH_3$ | 7-$CH_3$ | H | OH | |
| 2.11 | 4 | 7-$CH_3$ | 7-$CH_3$ | H | OH | |

Tabelle II (Fortsetzung)

| Verb.No. | m | $R_1$ | $R_2$ | $R_3$ | A | phys. Daten |
|---|---|---|---|---|---|---|
| 2.12 | 4 | 5-$CH_3$ | 5-$CH_3$ | H | OH | |
| 2.13 | 4 | 5-$CH_3$ | 6-$CH_3$ | 8-$CH_3$ | OH | |
| 2.14 | 4 | 8-$(CH_2)_2$-8 | | H | OH | |
| 2.15 | 3 | 7-$(CH_2)_2$-7 | | H | OH | |
| 2.16 | 4 | 8-$(CH_2)_2$-8 | | 6-$CH_3$ | OH | |
| 2.17 | 3 | 7-$CH_3$ | H | H | OH | |
| 2.18 | 3 | 7-$CH_3$ | 7-$CH_3$ | H | OH | |
| 2.19 | 3 | 5-$CH_3$ | 7-$CH_3$ | H | OH | |
| 2.20 | 3 | 6-$CH_3$ | 7-$CH_3$ | H | OH | |
| 2.21 | 3 | 6-$CH_3$ | 6-$CH_3$ | H | OH | |
| 2.22 | 3 | 6-$CH_3$ | H | H | OH | |
| 2.23 | 4 | 6-$CH_3$ | H | H | OH | |
| 2.24 | 4 | 6-$CH_3$ | 6-$CH_3$ | H | $OCH_3$ | Fp. 146-148°C |
| 2.25 | 4 | 6-$CH_3$ | 6-$CH_3$ | H | OH | Fp. 176°C |
| 2.26 | 4 | 7-$CH_3$ | H | H | $OCH_3$ | Fp. 138-140°C |

1) Verbindung 2.06 und 2.07 als Gemisch isoliert.

2) Verbindung 2.02 und 2.03 als Gemisch isoliert.

H.3. Verbindungen der Formel Ib

H.3.1. 2,8-Dimethyl-2-isopropyl-7,8,9,10-tetrahydro-3H,5H-imidazo[1′,2′:1,2]pyrrolo[3,4-b]chinolin-3,5-dion

13,0 g 2-(5-Isopropyl-5-methyl-1H-imidazol-5-on-2-yl)-6-methyl-chinolin-3-carbonsäure (erhältlich analog Beispiel H.2.2.) werden in 300 ml Dichlormethan mit 9,0 g Dicyclohexylcarbodiimid verrührt. Nach einigen Stunden bildet sich ein Kristallbrei. Man filtriert, dampft das Filtrat ein und kristallisiert den Rückstand aus Dichlormethan/Hexan um.

Man isoliert 11,0 g der Titelverbindung der Formel

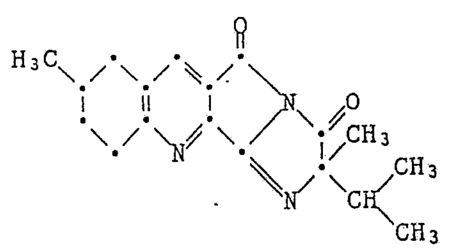

als Kristalle vom Smp. 162-165°C (Verbindung 3.01).

H.3.2. 2,7(9)-Dimethyl-2-isopropyl-7,8,9,10-tetrahydro-3H,5H-imidazo[1′,2′:1,2]pyrrolo[3,4-b]chinolin-3,5-dion

Analog zu Beispiel H.3.1. erhält man aus 13,0 g des Carbonsäuregemisches gemäss Beispiel H.2.3. 11,8 g der Titelverbindung als Gemisch der Verbindungen der Formel

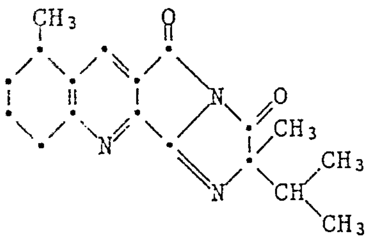

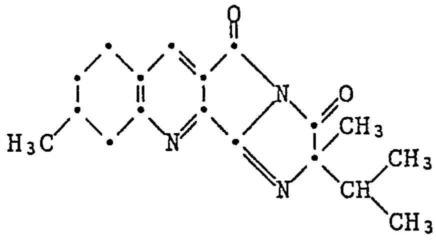

mit einem Smp. von 128-133° C (Verbindungen 3.02 und 3.03).

Analog zu den vorstehenden Beispielen H.3.1. bis H.3.2. sind die Verbindungen der Tabelle III herstellbar.

Verbindungen der Formel Ib

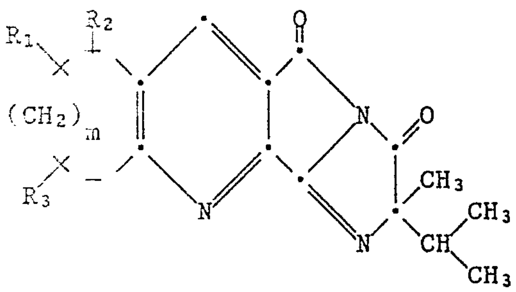

(Ib).

Tabelle III

| Verb.No. | m | R1 | R2 | R3 | phys. Daten |
|---|---|---|---|---|---|
| 3.01 | 4 | 8-$CH_3$ | H | H | Fp. 162-165°C |
| 3.02 | 4 | 7-$CH_3$ | H | H | Fp. 128-133°C[1)] |
| 3.03 | 4 | 9-$CH_3$ | H | H | Fp. 128-133°C[1)] |
| 3.04 | 4 | 10-$CH_3$ | H | H | |
| 3.05 | 4 | 7-$CH_3$ | 10-$CH_3$ | 10-$CH_3$ | |
| 3.06 | 3 | 7-$CH_3$ | H | H | |
| 3.07 | 3 | 8-$CH_3$ | H | H | |
| 3.08 | 4 | 10-$CH_3$ | 10-$CH_3$ | H | |
| 3.09 | 4 | 8-$CH_3$ | 10-$CH_3$ | H | |
| 3.10 | 4 | 7-$CH_3$ | 9-$CH_3$ | H | |
| 3.11 | 4 | 9-$CH_3$ | 9-$CH_3$ | H | |
| 3.12 | 4 | 7-$CH_3$ | 7-$CH_3$ | H | |
| 3.13 | 4 | 7-$CH_3$ | 8-$CH_3$ | 10-$CH_3$ | |
| 3.14 | 4 | 10-$(CH_2)_2$-10 | | H | |
| 3.15 | 3 | 9-$(CH_2)_2$-9 | | H | |
| 3.16 | 4 | 10-$(CH_2)_2$-10 | | 8-$CH_3$ | |
| 3.17 | 3 | 9-$CH_3$ | H | H | |
| 3.18 | 3 | 9-$CH_3$ | 9-$CH_3$ | H | |
| 3.19 | 3 | 7-$CH_3$ | 9-$CH_3$ | H | |
| 3.20 | 3 | 8-$CH_3$ | 9-$CH_3$ | H | |
| 3.21 | 3 | 8-$CH_3$ | 8-$CH_3$ | H | |
| 3.22 | 3 | 8-$CH_3$ | H | H | |
| 3.23 | 4 | 8-$CH_3$ | 8-$CH_3$ | H | Fp. 183-185°C |

[1)]Verbindung 3.02 und 3.03 als Gemisch isoliert.

H.4. Verbindungen der Formel Ic

H.4.1. Allgemeines Verfahren der säurekatalysierten Isomerisierung von Verbindungen der Formel

Zu einer Lösung von 0,5 g einer Verbindung der Formel Ib in 5 ml Acetanhydrid gibt man 50 mg Natriumacetat und erwärmt für 2 Stunden zum Sieden. Nach Abdestillieren des Lösungsmittels wird der Rückstand in Ether aufgenommen aus dem dann die umgelagerte Verbindung der Formel Ic isoliert werden kann.

H.4.2. Allgemeines Verfahren, ausgehend von Imiden der Formel V

Eine Lösung eines Imids der Formel V (herstellbar gemäss EP-A-0041634, EP-A-0212200, DE-A-3420271 oder GB-A-2174395) in Toluol wird in Gegenwart einer 1 bis 5 molaren Menge gepulverten NaOH am Wasserabscheider zum Sieden erhitzt.

Nach dem Erkalten wird das Reaktionsgemisch über Kieselgel filtriert, mit Essigsäureethylester nachgewaschen und die vereinigte Filtrate zur Trockene eingeengt.

Das so erhältliche Produkt wird gegebenenfalls zur Reinigung aus einem geeigneten Lösungsmittel umkristallisiert.

Analog zu den vorstehenden Beispielen H.4.1. bis H.4.2. sind die Verbindungen der Tabelle IV herstellbar.

Verbindungen der Formel Ic

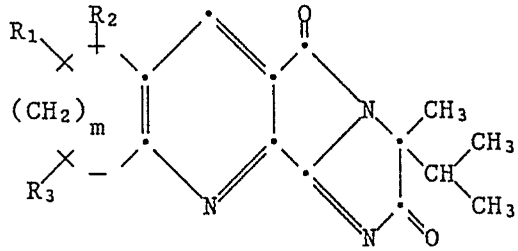

(Ic).

Tabelle IV

| Verb.No. | m | $R_1$ | $R_2$ | $R_3$ | phys. Daten |
|---|---|---|---|---|---|
| 4.01 | 4 | 8-$CH_3$ | H | H | |
| 4.02 | 4 | 7-$CH_3$ | H | H | |
| 4.03 | 4 | 9-$CH_3$ | H | H | |
| 4.04 | 4 | 10-$CH_3$ | H | H | |
| 4.05 | 4 | 7-$CH_3$ | 9-$CH_3$ | 9-$CH_3$ | |
| 4.06 | 3 | 7-$CH_3$ | H | H | |

Tabelle IV (Fortsetzung)

| Verb.No. | m | $R_1$ | $R_2$ | $R_3$ | phys. Daten |
|---|---|---|---|---|---|
| 4.07 | 3 | 8-$CH_3$ | H | H | |
| 4.08 | 4 | 10-$CH_3$ | 10-$CH_3$ | H | |
| 4.09 | 4 | 8-$CH_3$ | 10-$CH_3$ | H | |
| 4.10 | 4 | 7-$CH_3$ | 9-$CH_3$ | H | |
| 4.11 | 4 | 9-$CH_3$ | 9-$CH_3$ | H | |
| 4.12 | 4 | 7-$CH_3$ | 7-$CH_3$ | H | |
| 4.13 | 4 | 7-$CH_3$ | 8-$CH_3$ | 10-$CH_3$ | |
| 4.14 | 4 | 10-$(CH_2)_2$- | | H | |
| 4.15 | 3 | 9-$(CH_2)_2$- | | H | |
| 4.16 | 4 | 10-$(CH_2)_2$- | | 8-$CH_3$ | |
| 4.17 | 3 | 9-$CH_3$ | H | H | |
| 4.18 | 3 | 9-$CH_3$ | 9-$CH_3$ | H | |
| 4.19 | 3 | 7-$CH_3$ | 9-$CH_3$ | H | |
| 4.20 | 3 | 8-$CH_3$ | 9-$CH_3$ | H | |
| 4.21 | 3 | 8-$CH_3$ | 8-$CH_3$ | H | |
| 4.22 | 3 | 8-$CH_3$ | H | H | |
| 4.23 | 4 | 6-$CH_3$ | 6-$CH_3$ | H | |

## F.1. Formulierungsbeispiele

| a) Spritzpulver | a) | b) | c) |
|---|---|---|---|
| Wirkstoff gemäss Tabelle 2, 3 oder 4 | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyethylenglykol-ether (7-8 Mol EO) | - | 2 % | 2 % |
| Hochdisperse Kieselsäure | 5 % | 27 % | 27 % |
| Kaolin | 67 % | - | 59,5 % |

a) Spritzpulver

| | a) | b) | c) |
|---|---|---|---|
| Wirkstoff gemäss Tabelle 2, 3 oder 4 | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyethylenglykolether (7-8 Mol EO) | - | 2 % | 2 % |
| Hochdisperse Kieselsäure | 5 % | 27 % | 27 % |
| Kaolin | 67 % | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsions-Konzentrat

| | a) | b) |
|---|---|---|
| Wirkstoff gemäss Tabelle 2, 3 oder 4 | 10 % | 1 % |
| Octylphenolpolyethylenglykolether (4-5 Mol EO) | 3 % | 3 % |
| Ca-Dodecylbenzolsulfonat | 3 % | 3 % |
| Ricinusölpolyglykolether (36 Mol EO) | 4 % | 4 % |
| Cyclohexanon | 30 % | 10 % |
| Xylolgemisch | 50 % | 79 % |

Aus diesen Konzentraten können durch Verdünnen mit Wasser Emulsionen jeder gewünschten Konzentration hergestellt werden.

c) Stäubemittel

| | a) | b) |
|---|---|---|
| Wirkstoff gemäss Tabelle 2, 3 oder 4 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer

geeigneten Mühle vermahlen wird.

d) Extruder Granulat

| | a) | b) |
|---|---|---|
| Wirkstoff gemäss Tabelle 2, 3 oder 4 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff gemäss Tabelle 2, 3 oder 4 | 3 % |
| Polyethylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff gemäss Tabelle 2, 3 oder 4 | 40 % | 5 % |
| Ethylenglykol | 10 % | 10 % |
| Nonylphenol-polyethylenglykolether (15 Mol EO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

B. Biologische Beispiele:

B.1. Herbizidwirkung vor dem Auflaufen der Pflanzen

Kunststofftöpfe werden mit expandiertem Vermiculit (Dichte: 0,135 g/cm³, Wasserabsorptionsvermögen: 0,565 1/1) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Kresse, Agrostis tenuis, Stellaria media und Digitaria sanguinalis. Die Versuchsgefässe werden anschliessend in einer Klimakammer bei 20° C, einer Beleuchtung von ca. 20 kLux und einer relativen Luftfeuchtigkeit von 70 % gehalten. Während der Keimphase von 4 bis 6 Tagen werden die Töpfe zur Erhöhung der örtlichen Luftfeuchtigkeit mit lichtdurchlässigem Material abgedeckt und mit deinoisiertem Wasser begossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen nach dem in Beispiel B.2. gegebenen Massstab bewertet.

Die Resultate sind in der Tabelle 5 zusammengefasst:

| Verb. No. | Kresse | Agrostis | Stellaria | Digitaria |
|---|---|---|---|---|
| 2.01 | 2 | 2 | 2 | 2 |
| 2.02[1)] | 2 | 2 | 2 | 2 |
| 2.03[1)] | 2 | 2 | 2 | 2 |
| 2.06[2)] | 2 | 2 | 2 | 2 |
| 2.07[2)] | 2 | 2 | 2 | 2 |
| 3.01 | 2 | 2 | 2 | 2 |

[1)] Verbindungen 2.02 und 2.03 werden als Gemisch gemäss Herstellungsbeispiel H.2.3. getestet.
[2)] Verbindungen 2.06 und 2.07 werden als Gemisch gemäss Herstellungsbeispiel H.2.2. getestet.

B.2. Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25 %igen Emulsionskonzentrat behandelt. Es werden Konzentrationen von 2000-500 g Wirksubstanz/Hektar angewendet. Die Saatschalen werden im Gewächshaus bei 22-25° C und 50-70 % relativer Luftfeuchtigkeit gehalten und der Versuch nach 3 Wochen ausgewertet und die Resultate nach folgender Notenskala ausgewertet.

1 Pflanze hat nicht gekeimt oder ist eingegangen
2-3 sehr starke Schäden
4 starke Schäden
5 mittlere Schäden, die Pflanzen sind verkümmert
6 Schäden, die Pflanze kann regenerieren
7-8 leichte Schäden
9 normales Wachstum, wie das unbehandelter Pflanzen

Boniturnoten von 1 bis 4 (insbesondere von 1 bis 3) weisen auf eine gute bis sehr gute Herbizidwirkung hin. Boniturnoten von 6 bis 9 (insbesondere 7 bis 9) weisen auf eine gute Toleranz (insbesondere bei Kulturpflanzen) hin.

In diesem Test zeigen die Verbindungen 2.01, 2.02, 3.01 und 3.02 bei Aufwandmengen von 500 bis 2000 [g/ha] gute bis sehr gute Herbizidwirkung bei folgenden Testpflanzen: Avena fatua, Bromus tectorum, Lolium perenne, Alopecurus myos., Digitaria sang., Echinochloa crus galli, Sorghum halep., Abutilon, Amaranthus ret., Chenopodium Sp., Solanum nigrum, Ipomoea, Sinapis, Stellaria, Chrysanthe. leuc., Galium aparine und Veronica Sp.; Soja wird bei diesen Aufwandmengen nicht oder nur in geringem Masse im Wachstum beeinflusst (gute Toleranz).

B.3. Post-emergente Herbizid-Wirkung (Kontaktherbizid)

Eine Anzahl Unkräuter, sowohl monokotyle wie dikotyle, werden nach dem Auflaufen (im 4- bis 6-Blattstadium) mit einer wässrigen Wirkstoffdispersion in einer Dosierung von 2000-500 g Wirksubstanz pro Hektar auf die Pflanzen gespritzt und diese bei 24°-26° C und 45-60 % relativer Luftfeuchtigkeit gehalten. 15 Tage nach der Behandlung wird der Versuch nach dem in Beispiel B.2. gegebenen Massstab bewertet.

In diesem Test zeigen die Verbindungen 2.01, 2.02, 2.06, 3.01 und 3.02 bei Aufwandmengen von 500 bis 2000 [g/ha] gute bis sehr gute Herbizidwirkung bei folgenden Testpflanzen: Avena fatua, Bromus tectorum, Lolium perenne, Alopecurus myos., Digitaria sang., Echinochloa crus galli, Sorghum halep., Abutilon, Xanthium Sp., Amaranthus ret., Chenopodium Sp., Solanum nigrum, Sinapis, Stellaria, Chrysanthe. leuc., Galium aparine und Veronica Sp.; Soja und Baumwolle werden bei diesen Aufwandmengen nicht oder nur in geringem Masse im Wachstum beeinflusst (gute Toleranz).

B.4. Wuchshemmung bei Gräsern, Postemergente Applikation

Eine Mischung von Gräsern (z.B. Poa, Festuca, Lolium, Bromus, Cynosurus) werden in 15 cm - Kunststofftöpfen mit steriler Landerde angesät und im Gewächshaus bei einer Tagestemperatur von 21° C und einer Nachttemperatur von 17° C angezogen. Die Beleuchtungsdauer beträgt 13,5 Stunden/Tag bei einer

Lichtintensität von mind. 7000 Lux. Nach dem Ablauf werden die Pflanzen wöchentlich auf ca. 6 cm Höhe zurückgeschnitten. Ca. 42 Tage nach der Saat und 1 Tag nach dem letzten Schnitt erfolgt die Applikation mit 60-500 g Wirkstoff/ha als wässrige Spritzbrühe. Die Wasseraufwandmenge ist ca. 500 l/ha.

20 Tage nach der Behandlung wird der Versuch ausgewertet. Hierbei wird die Höhe des Neuzuwachses gemessen und in % Hemmung zum Durchschnitt der unbehandelten Kontrollen dargestellt. Die nekrotischen Schäden sind in % zur gesamten Blattfläche aufgeführt.

Die Resultate sind in Tabelle 6 zusammengestellt:
(WH = Wuchshemmung in %
Nekr = nekrotische Schäden in %)

Tabelle 6

| Verb.No. | Aufwandmenge 60 [g/ha] % WH | 60 [g/ha] % Nekr | 250 [g/ha] % WH | 250 [g/ha] % Nekr | 500 [g/ha] % WH | 500 [g/ha] % Nekr |
|---|---|---|---|---|---|---|
| 2.01 | 33 | 10 | 90 | 15 | 90 | 25 |
| 2.02 | 14 | 5 | 84 | 15 | 90 | 20 |
| 2.04 | 0 | - | 62 | - | 87 | 20 |
| 2.06 | 71 | 15 | 87 | 15 | 97 | 35 |
| 2.12 | 11 | 5 | 49 | 5 | 84 | 5 |
| 2.17 | 20 | 5 | 90 | 20 | 94 | 30 |
| 3.01 | 46 | 15 | 78 | 15 | 87 | 15 |
| 3.02 | 4 | 5 | 78 | 15 | 87 | 20 |
| 3.03 | 1 | - | 68 | 5 | 84 | 10 |

B.5. Wuchshemmung Mais, preemergente Applikation

Mais der Sorte Blizzard G188 werden in 15 cm - Kunststofftöpfen mit steriler gesiebter Landerde angesät und im Gewächshaus bei einer Tagestemperatur von 22° C und einer Nachttemperatur von 19° C aufgestellt. Die Beleuchtungsdauer beträgt mindestens 13,5 Stunden/Tag bei einer Lichtintensität von mindestens 7000 Lux. 1 Tag nach der Saat erfolgt die Applikation mit 60-500 g Wirkstoff/ha, in der Regel 25 %ig formuliert und in wässriger Spritzbrühe. Die Wasseraufwandmenge ist ca. 200 l/ha.

16 Tage nach der Behandlung wird der Versuch ausgewertet. Hierbei wird die Höhe des Neuzuwachses gemessen und in % Hemmung zum Durchschnitt der unbehandelten Kontrollen dargestellt.

Die Resultate sind in Tabelle 7 zusammengestellt:
(WH = Wuchshemmung in %)

Tabelle 7

| Verb.No. | Aufwandmenge 60 [g/ha] WH | 125 [g/ha] WH | 250 [g/ha] WH | 500 [g/ha] WH |
|---|---|---|---|---|
| 2.01 | 5 | 10 | 48 | 69 |
| 2.02 | 2 | 5 | 38 | 43 |
| 2.04 | 0 | 0 | 7 | 55 |
| 2.12 | 0 | 0 | 2 | 2 |
| 3.01 | 0 | 0 | 10 | 62 |
| 3.02 | 2 | 4 | 5 | 5 |

B.6. Wuchshemmung bei Raps, postemergente Applikation

Winterraps der Sorte Bienvenu wird in 15 cm - Kunststofftöpfen mit steriler Landerde angesät und im Gewächshaus/Klimakammer bei folgenden Temperaturen angezogen:

- 7 Tage Tagestemperatur 22° C. Nachttemperatur 19° C
- 17 Tage Tagestemperatur 10° C. Nachttemperatur 5° C
- 7 Tage Tagestemperatur 15° C. Nachttemperatur 10° C
- 4 Tage Tagestemperatur 20° C. Nachttemperatur 10° C

Die Beleuchtungsdauer beträgt mindestens 13,5 Stunden/Tag bei einer Lichtintensität von mindestens 7000 Lux. Pro Topf werden 3 Pflanzen stehengelassen.

Ca. 35 Tage nach der Saat erfolgt die Applikation mit 30-500 g Wirkstoff/ha, in der Regel 25 %ig formuliert und in wässriger Spritzbrühe. Die Wasseraufwandmenge beträgt ca. 200 l/ha.

28 Tage nach der Behandlung wird der Versuch ausgewertet. Hierbei wird die Höhe des Neuzuwachses gemessen und in % Hemmung zum Durchschnitt der unbehandelten Kontrollen dargestellt. Die nekrotischen

Schäden sind in % zur gesamten Blattfläche aufgeführt.

Die Ergebnisse sind in Tabelle 8 zusammengestellt:
(WH = Wuchshemmung in %
Nekr = nekrotische Schäden in %)

Tabelle 8

| | Aufwandmenge | | | | | | | | | |
|---|---|---|---|---|---|---|---|---|---|---|
| Verb.No. | 30 [g/ha] | | 60 [g/ha] | | 125 [g/ha] | | 250 [g/ha] | | 500 [g/ha] | |
| | WH | Nekr | WH | Nekr | WH | Nekr | WH | Nekr | WH | Nekr |
| 2.01 | 5 | - | 7 | - | 20 | - | 52 | - | 70 | 15 |
| 2.02 | 0 | - | 7 | - | 7 | - | 32 | - | 45 | - |
| 2.04 | 0 | - | 5 | - | 22 | - | 47 | - | 47 | 15 |
| 2.06 | 0 | - | 13 | - | 42 | - | 47 | - | 60 | 10 |
| 2.12 | 0 | - | 0 | - | 20 | 5 | 40 | - | 52 | 10 |
| 3.01 | 7 | - | 10 | - | 5 | - | 45 | - | 57 | - |
| 3.02 | 0 | - | 0 | - | 0 | - | 37 | 5 | 45 | 5 |
| 3.03 | 0 | - | 2 | - | 20 | - | 55 | - | 55 | 15 |

B.9. Wuchshemmung bei Baumwolle, postemergente Applikation

Baumwolle der Sorte Acala wird in 13 cm - Kunststofftöpfen mit 45 % steriler Landerde, 45 % Torfsubstrat und 10 % Zonolit angesät und im Gewächshaus bei einer Tagestemperatur von mindestens 25° C und einer Nachttemperatur von mindestens 20° C angezogen. Pro Topf wird 1 Pflanze stehengelassen.
Ca. 50 Tage nach der Saat und mit 5 entwickelten Blättern erfolgt die Applikation mit 60-1000 g Wirkstoff/ha, in der Regel 25 %ig formuliert und in wässriger Spritzbrühe. Die Wasseraufwandmenge beträgt ca. 200 l/ha. 28 Tage nach der Behandlung wird der Versuch ausgewertet. Hierbei wird die Höhe des Neuzuwachses gemessen und in % Hemmung zum Durchschnitt der unbehandelten Kontrollen dargestellt. Die nekrotischen Schäden sind in % zur gesamten Blattfläche aufgeführt.

Die Ergebnisse sind in Tabelle 9 zusammengestellt:
(WH = Wuchshemmung in %,
Nekr = nekrotische Schäden in %)

Tabelle 9

| | Aufwandmenge | | | | | | | | | |
|---|---|---|---|---|---|---|---|---|---|---|
| Verb.No. | 60 [g/ha] | | 125 [g/ha] | | 250 [g/ha] | | 500 [g/ha] | | 1000 [g/ha] | |
| | WH | Nekr | WH | Nekr | WH | Nekr | WH | Nekr | WH | Nekr |
| 2.01 | 0 | - | 7 | - | 3 | - | 27 | - | 40 | - |
| 2.02 | 7 | - | 7 | - | 30 | - | 40 | - | 40 | - |
| 2.04 | 0 | - | 7 | - | 7 | - | - | - | 27 | - |
| 2.06 | 12 | - | 12 | - | 12 | - | 40 | - | 60 | - |
| 2.12 | 0 | - | 0 | - | 17 | - | 27 | - | 30 | - |
| 2.17 | 17 | - | 23 | - | 27 | - | 37 | - | 77 | 5 |
| 3.01 | 17 | - | 17 | - | 17 | - | 17 | - | 20 | - |
| 3.02 | 0 | - | 10 | - | 17 | - | 17 | - | 40 | - |
| 3.03 | 0 | - | 0 | - | 23 | - | 37 | - | 53 | - |

**Patentansprüche**

1. Verbindungen der Formel I,

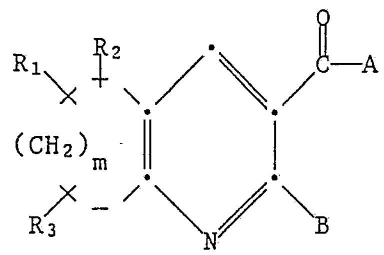

(I),

worin $R_1$ $C_1$-$C_4$-Alkyl;
$R_2$ und $R_3$ unabhängig voneinander Wasserstoff; oder $C_1$-$C_4$-Alkyl;
$R_1$ und $R_2$ ausserdem gemeinsam einen geminal gebundenen $-(CH_2)_n-$Ring;
m 3 oder 4;
n 2 bis 5;
A OH, $O^{\ominus}M^{\oplus}$ oder $C_1$-$C_4$-Alkoxy;
$M^{\oplus}$ ein Kationäquivalent eines Alkali- oder Erdalkalimetalls oder einer stickstoffhaltigen Base;
B ein Imidazolon der Formel

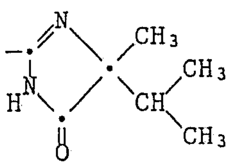

;

oder
A und B gemeinsam ein anelliertes Imidazolon der Formel

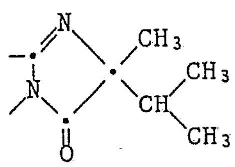

bedeutet,
sowie Salze von Verbindungen der Formel I mit Säuren, Basen und Komplexbildnern.

2. Verbindungen der Formel I gemäss Anspruch 1 worin
$R_1$ $C_1$-$C_3$-Alkyl;
$R_2$ und $R_3$ unabhängig voneinander Wasserstoff oder $C_1$-$C_3$-Alkyl;
$R_1$ und $R_2$ ausserdem gemeinsam einen geminal gebundenen $-(CH_2)_n-$Ring;
m 3 oder 4;
n 2, 4 oder 5;
A OH, $O^{\ominus}M^{\oplus}$ oder $C_1$-$C_3$-Alkoxy;
B ein Imidazolon der Formel

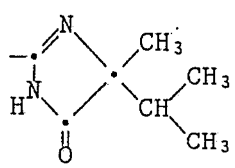

;

oder
A und B gemeinsam ein anelliertes Imidazolon der Formel

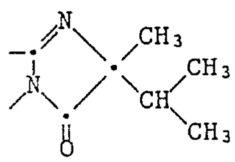

bedeutet.

3. Verbindung der Formel I, gemäss Anspruch 2, worin
$R_1$ Methyl oder Ethyl;
$R_2$ und $R_3$ unabhängig voneinander Wasserstoff; Methyl oder Ethyl;
$R_1$ und $R_2$ ausserdem gemeinsam einen geminal gebundenen $-(CH_2)_n-$Ring mit
n = 2 oder 4
bedeutet und
die Reste m, A und B wie zuvor definiert sind.

4. 7H-5,6-Dihydro-2-(5-isopropyl-5-methyl-1H-imidazol-4-on-2-yl)-5-methyl-1-pyrindin-3-carbonsäure,
7H-5,6-Dihydro-2-(5-isopropyl-5-methyl-1H-imidazol-4-on-2-yl)-6-methyl-1-pyrindin-3-carbonsäure,
2-(5-Isopropyl-5-methyl-1H-imidazol-4-on-2-yl)-5-methyl-5,6,7,8-tetrahydrochinolin-3-carbonsäure,
2-(5-Isopropyl-5-methyl-1H-imidazol-4-on-2-yl)-7-methyl-5,6,7,8-tetrahydrochinolin-3-carbonsäure,
2-(5-Isopropyl-5-methyl-1H-imidazol-4-on-2-yl)-8-methyl-5,6,7,8-tetrahydrochinolin-3-carbonsäure,
2-(5-Isopropyl-5-methyl-1H-imidazol-4-on-2-yl)-5,6,7,8-tetrahydro-5,7,7-trimethylchinolin-3-carbonsäure,
2-(5-Isopropyl-5-methyl-1H-imidazol-4-on-2-yl)-6-methyl-5,6,7,8-tetrahydrochinolin-3-carbonsäure,
6,6-Dimethyl-2-(5-isopropyl-5-methyl-1H-imidazol-4-on-2-yl)-5,6,7,8-tetrahydrochinolin-3-carbonsäure,
6,6-Dimethyl-2-(5-isopropyl-5-methyl-1H-imidazol-4-on-2-yl)-3-methoxycarbonyl-5,6,7,8-tetrahydrochinolin,
2-(5-Isopropyl-5-methyl-1H-imidazol-4-on-2-yl)-3-methoxycarbonyl-7-methyl-5,6,7,8-tetrahydrochinolin,
2,7-Dimethyl-2-isopropyl-7,8,9,10-tetrahydro-3H,5H-imidazo[1′,2′:1,2]pyrrolo[3,4-b]chinolin-3,5-dion,
2,8-Dimethyl-2-isopropyl-7,8,9,10-tetrahydro-3H,5H-imidazo[1′,2′:1,2]pyrrolo[3,4-b]chinolin-3,5-dion
2-Isopropyl-7,8,9,10-tetrahydro-2,8,8-trimethyl-3H,5H-imidazo[1′,2′:1,2]pyrrolo[3,4-b]chinolin-3,5-dion
oder
2,9-Dimethyl-2-isopropyl-7,8,9,10-tetrahydro-3H,5H-imidazo[1′,2′:1,2]pyrrolo[3,4-b]chinolin-3,5-dion gemäss Anspruch 1.

5. Verfahren zur Herstellung von Verbindungen der Formel I, gemäss Anspruch 1 dadurch gekennzeichnet, dass man

a) einen Diester der Formel II

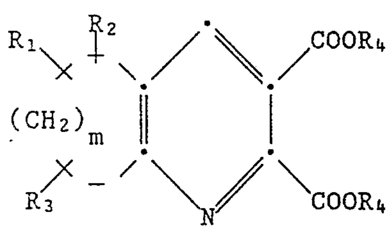

(II)

worin $R_1$, $R_2$, $R_3$ und m wie zuvor definiert sind und die Reste $R_4$ unabhängig voneinander für $C_1$-$C_6$-Alkyl stehen mit einem 2-Amino-2,3-dimethylbuttersäureamid der Formel III

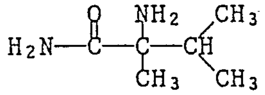

(III)

in Gegenwart einer Base zu einer Verbindung der Formel Ia

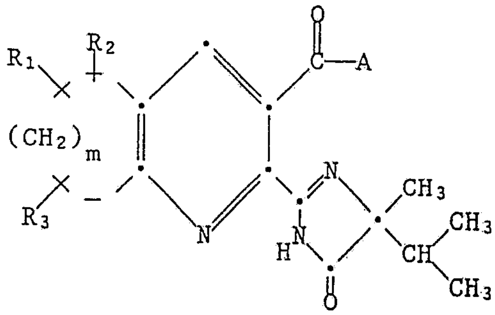

(Ia)

worin A für OH steht und die Reste X, $R_1$, $R_2$, $R_3$ und m wie zuvor definiert sind, umsetzt, oder

b) eine Verbindung der Formel Ia

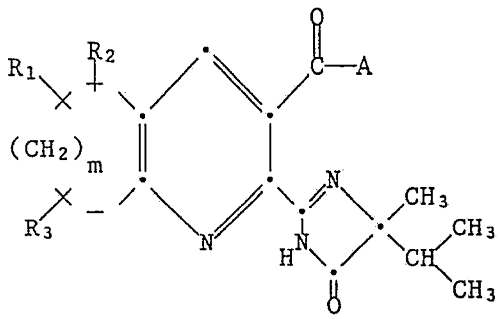

(Ia)

worin $R_1$, $R_2$, $R_3$ und m wie zuvor definiert sind und A für OH steht in Gegenwart eines wasserbindenden bzw. wasserabscheidenden Mittels zu Ib

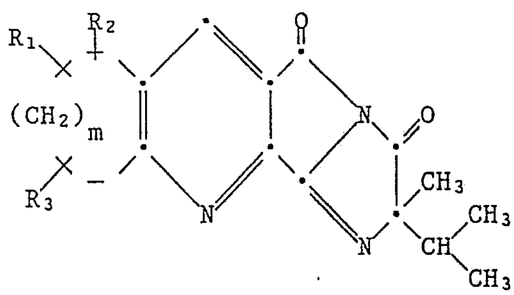

(Ib)

worin $R_1$, $R_2$, $R_3$ und m wie zuvor definiert sind, cyclisiert, oder

c) ein Säureanhydrid der Formel IV

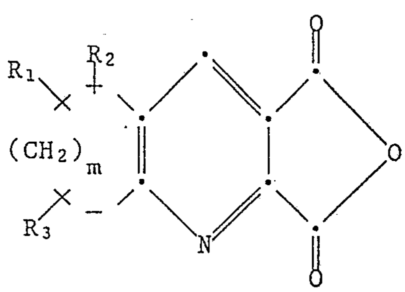

(IV)

worin $R_1$, $R_2$, $R_3$ und m wie zuvor definiert sind, mit 2-Amino-2,3-dimethylbuttersäureamid der Formel III

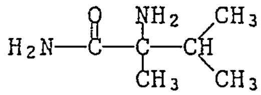

(III)

zu einem Imid der Formel V

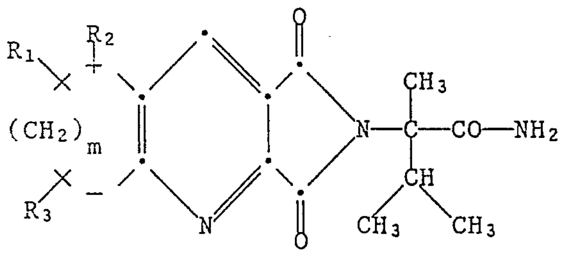

(V)

umsetzt und das so erhältliche Imid der Formel V in Gegenwart einer Base zu Ic

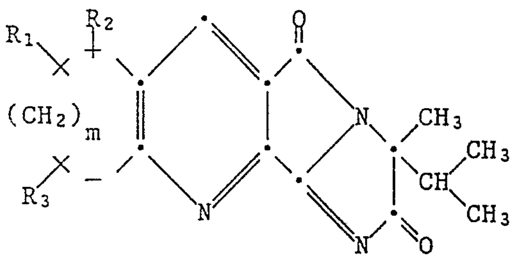

(Ic)

worin $R_1$, $R_2$, $R_3$ und m wie zuvor definiert sind, cyclisiert, oder

d) eine Verbindung der Formel Ib

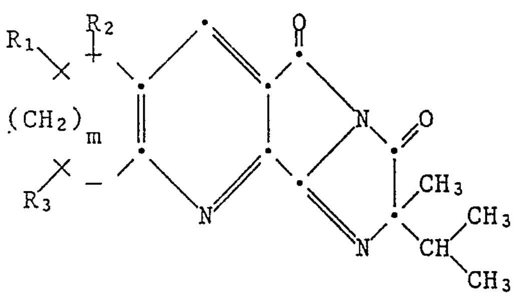

(Ib)

worin $R_1$, $R_2$, $R_3$ und m wie zuvor definiert sind zu Ic

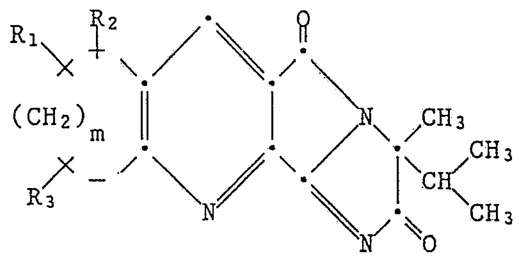

(Ic)

worin $R_1$, $R_2$, $R_3$ und m wie zuvor definiert sind, isomerisiert, oder

e) eine Verbindung der Formel Ib

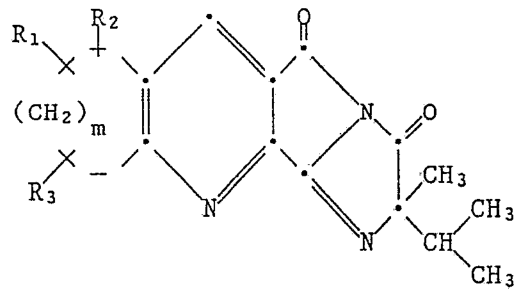

(Ib)

oder eine Verbindung der Formel Ic

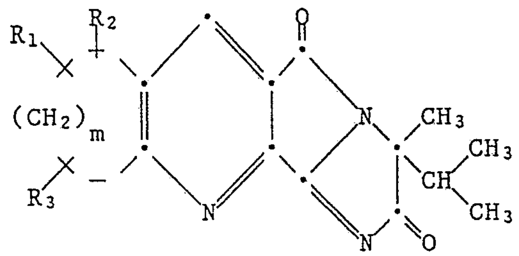

(Ic)

in welchen die Reste $R_1$, $R_2$, $R_3$ und m wie zuvor definiert sind, mit einem Alkohol der Formel VI

H A VI,

worin A für $C_1$-$C_4$-Alkoxy steht, gewünschtenfalls in Gegenwart einer Säure oder Base zu einer Verbindung der Formel Ia

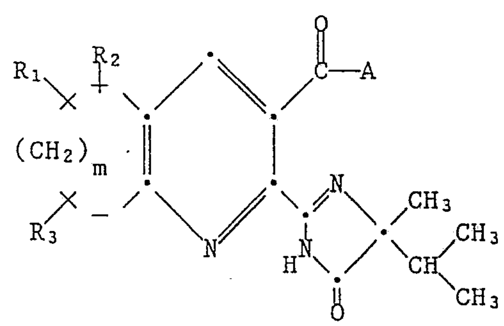

(Ia)

worin $R_1$, $R_2$, $R_3$ und m wie zuvor definiert sind und A für $C_1$-$C_4$-Alkoxy steht, umsetzt, oder

f) einen Diester der Formel II

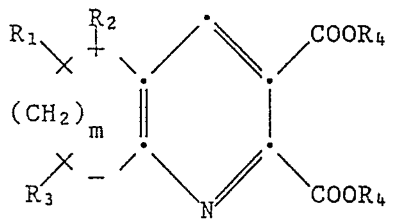

(II)

in dem $R_1$, $R_2$, $R_3$ und m wie zuvor definiert sind und $R_4$ für $C_1$-$C_4$-Alkyl steht, unter nicht hydrolisierenden Bedingungen und in Gegenwart einer Base mit 2-Amino-2,3-dimethylbuttersäureamid zu einer Verbindung der Formel Ia

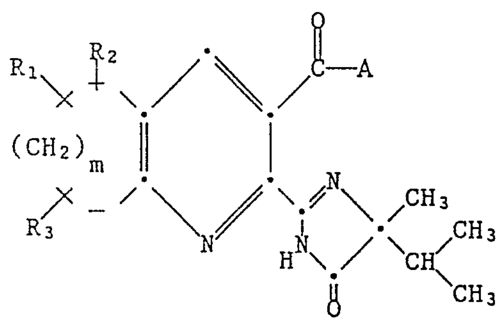

(Ia)

worin $R_1$, $R_2$, $R_3$ und m wie zuvor definiert sind und A für $C_1$-$C_4$-Alkoxy steht, umsetzt, oder

g) eine Verbindung der Formel Ia

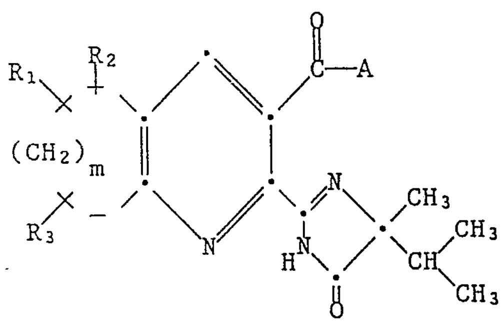

(Ia)

in der die Reste $R_1$, $R_2$, $R_3$ und m wie zuvor definiert sind und A für OH oder $C_1$-$C_4$-Alkoxy steht mit einer äquivalenten Menge einer Verbindung der Formel VII

$OH^{\ominus}M^{\oplus}$ (VII)

in der $M^{\oplus}$ für einen Kationenäquivalent eines Alkali- oder Erdalkalimetalls oder eine quaternäre stickstoffhaltige Base steht, zu einer Verbindung der Formel Ia'

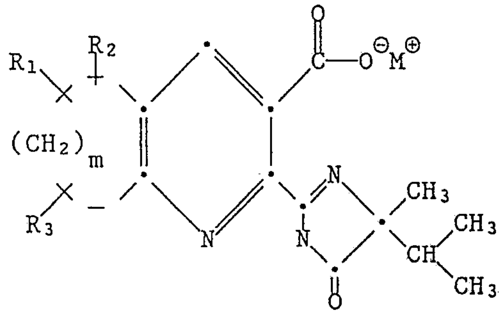

(Ia')

worin $R_1$, $R_2$, $R_3$ und m wie zuvor definiert sind und $M^{\oplus}$ für ein Kationenäquivalent eines Alkali- oder Erdalkalimetalls oder einer stickstoffhaltigen Base steht, umsetzt, oder

h) eine Verbindung der Formel Ia, in der A für OH steht mit einem Alkali-oder Erdalkalimetall, einem Alkali oder Erdalkalisalz oder der stickstoffhaltigen Base direkt zu einem Salz der Formel Ia'

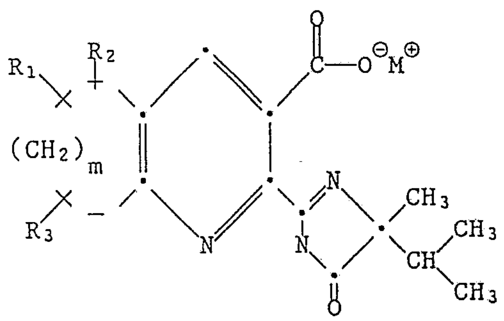

(Ia')

worin $R_1$, $R_2$, $R_3$ und m wie zuvor definiert sind und $M^{\oplus}$ für ein Kationenäquivalent eines Alkali- oder Erdalkalimetalls oder einer stickstoffhaltigen Base steht, umsetzt.

6. Verbindungen der Formel II

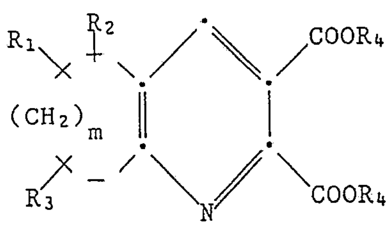

(II)

worin
$R_1$ $C_1$-$C_4$-Alkyl;
$R_2$ und $R_3$ unabhängig voneinander Wasserstoff; oder $C_1$-$C_4$-Alkyl;
$R_1$ und $R_2$ ausserdem gemeinsam einen geminal gebundenen $-(CH_2)_n-$Ring;
m 3 oder 4;
n 2 bis 5; und
$R_4$ $C_1$-$C_4$-Alkyl bedeutet oder
Dicarbonsäuren der Formel XII

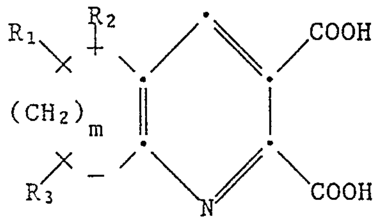

(XII),

worin
$R_1$ $C_1$-$C_4$-Alkyl;
$R_2$ und $R_3$ unabhängig voneinander Wasserstoff; oder $C_1$-$C_4$-Alkyl;
$R_1$ und $R_2$ ausserdem gemeinsam einen geminal gebundenen $-(CH_2)_{\overline{n}}$-Ring;
m 3 oder 4; und
n 2 bis 5 bedeutet oder
Anhydride der Formel IV

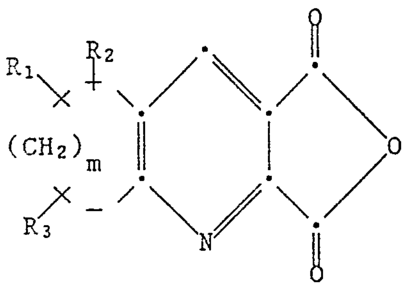

(IV),

worin
$R_1$ $C_1$-$C_4$-Alkyl;
$R_2$ und $R_3$ unabhängig voneinander Wasserstoff; oder $C_1$-$C_4$-Alkyl;
$R_1$ und $R_2$ ausserdem gemeinsam einen geminal gebundenen $-(CH_2)_{\overline{n}}$-Ring;
m 3 oder 4; und
n 2 bis 5 bedeutet oder
Imide der Formel V

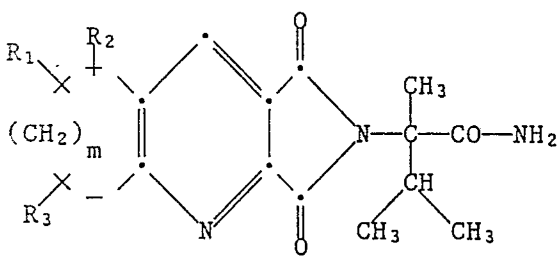

(V)

worin
$R_1$ $C_1$-$C_4$-Alkyl;
$R_2$ und $R_3$ unabhängig voneinander Wasserstoff; oder $C_1$-$C_4$-Alkyl;
$R_1$ und $R_2$ ausserdem gemeinsam einen geminal gebundenen $-(CH_2)_{\overline{n}}$-Ring;
m 3 oder 4; und
n 2 bis 5 bedeutet.

7. Verfahren zur Herstellung der Zwischenprodukte gemäss Anspruch 6, dadurch gekennzeichnet, dass man

a) ein Enamin der Formel VIII

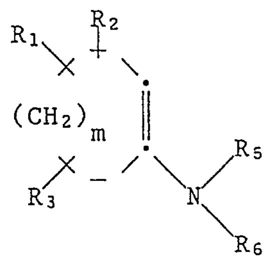

(VIII)

worin $R_5$ und $R_6$ unabhängig voneinander für $C_1$-$C_4$-Alkyl oder $R_5$ und $R_6$ gemeinsam eine, gewünschtenfalls einmal durch Sauerstoff oder N-$R_7$ (mit $R_7$ = Wasserstoff oder $C_1$-$C_4$-Alkyl) unterbrochene $-(CH_2)_p-$Kette bedeutet mit p = 4-6 und $R_1$, $R_2$, $R_3$ und m wie zuvor definiert sind, mit einem Diester der Formel IX

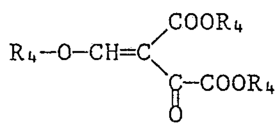

(IX),

worin $R_4$ für $C_1$-$C_4$-Alkyl steht umsetzt, und das aus VIII und IX erhältliche Monokondensationsprodukt in einem zweiten Reaktionsschritt mit Ammoniak zu einem Diester der Formel II cyclisiert, oder

b) im β-Hydroxyketon der Formel X

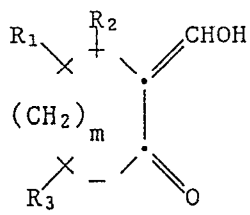

(X)

worin $R_1$, $R_2$, $R_3$ und m wie zuvor definiert sind mit einem Aminofumarat XI

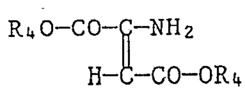

(XI)

worin $R_4$ wie zuvor definiert ist zu einem Diester der Formel II kondensiert, oder

c) einen Ester der Formel II

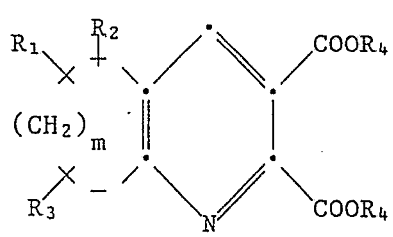

(II),

worin $R_1$, $R_2$, $R_3$ und m wie zuvor definiert sind und $R_4$ $C_1$-$C_4$-Alkyl bedeutet zu einer Dicarbonsäure der Formel XII verseift, oder

d) einen Ester der Formel II

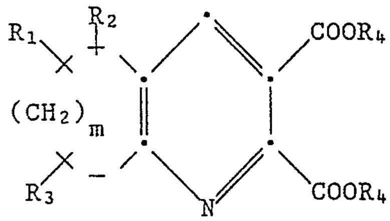

(II),

worin $R_1$, $R_2$, $R_3$ und m wie zuvor definiert sind und $R_4$ $C_1$-$C_4$-Alkyl bedeutet, zu einer Dicarbonsäure der Formel XII

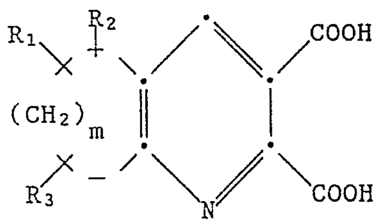

(XII),

worin $R_1$, $R_2$, $R_3$ und m wie zuvor definiert sind, verseift und anschliessend mit wasserabspaltenden Mitteln zu einem Anhydrid der Formel IV cyclisiert, oder

e) ein Anhydrid der Formel IV

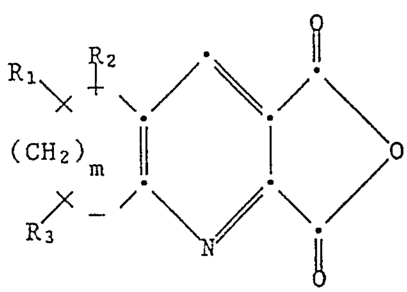

(IV),

worin $R_1$, $R_2$, $R_3$ und m wie zuvor definiert sind, mit 2-Amino-2,3-dimethylbuttersäureamid III

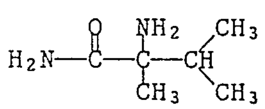

(III)

zu einem Imid der Formel V umsetzt.

8. Herbizides oder wuchsregulatorisches Mittel, enthaltend als Aktivsubstanz eine Verbindung der Formel I gemäss einem der Ansprüche 1 bis 4, neben weiteren Hilfs- und/oder Trägerstoffen.

9. Verfahren zu Bekämpfung unerwünschten Pflanzenwuchses, dadurch gekennzeichnet, dass man eine Verbindung der Formel I gemäss einem der Ansprüche 1 bis 4 oder ein herbizides Mittel gemäss Anspruch 8 auf die zu bekämpfende Pflanze oder deren Lebensraum einwirken lässt.

10. Verfahren zur Beeinflussung des vegetativen Wachstums von Pflanzen, dadurch gekennzeichnet, dass man eine pflanzenregulatorisch wirksame Menge einer Verbindung der Formel I gemäss einem der Ansprüche 1 bis 4 oder eines wuchsregulatorischen Mittels gemäss Anspruch 8 auf die Pflanze oder deren Lebensraum einwirken lässt.

11. Saatgut, erhältlich durch die Behandlung mit einer herbizid oder pflanzenwuchsregulatorisch wirksamen Menge einer Verbindung der Formel I gemäss einem der Ansprüche 1 bis 4 oder einer herbizid

oder pflanzenwuchsregulatorisch wirksamen Menge eines Mittels gemäss Anspruch 8.

**Patentansprüche für folgenden Vertragsstaat: ES**

1. Verfahren zur Herstellung von Verbindungen der Formel I

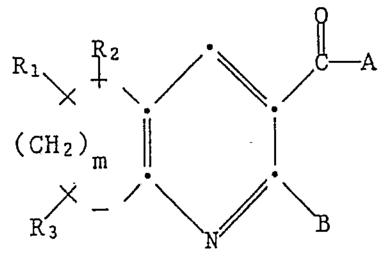

(I),

worin
$R_1$ $C_1$-$C_4$-Alkyl;
$R_2$ und $R_3$ unabhängig voneinander Wasserstoff; oder $C_1$-$C_4$-Alkyl;
$R_1$ und $R_2$ ausserdem gemeinsam einen geminal gebundenen $-(CH_2)_n-$Ring;
m 3 oder 4;
n 2 bis 5;
A OH, $O^{\ominus}M^{\oplus}$ oder $C_1$-$C_4$-Alkoxy;
$M^{\oplus}$ ein Kationäquivalent eines Alkali- oder Erdalkalimetalls oder einer stickstoffhaltigen Base;
B ein Imidazolon der Formel

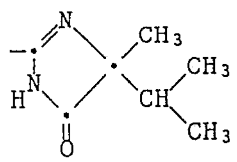

;

oder
A und B gemeinsam ein anelliertes Imidazolon der Formel

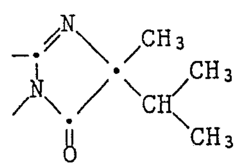

bedeutet, dadurch gekennzeichnet, dass man
a) einen Diester der Formel II

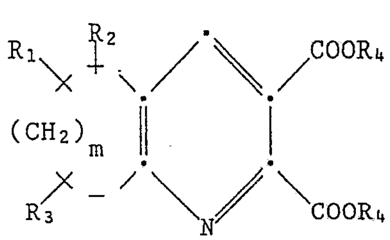

(II)

worin $R_1$, $R_2$, $R_3$ und m wie zuvor definiert sind und die Reste $R_4$ unabhängig voneinander für

$C_1$-$C_6$-Alkyl stehen mit einem 2-Amino-2,3-dimethylbuttersäureamid der Formel III

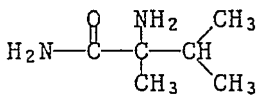

(III)

in Gegenwart einer Base zu einer Verbindung der Formel Ia

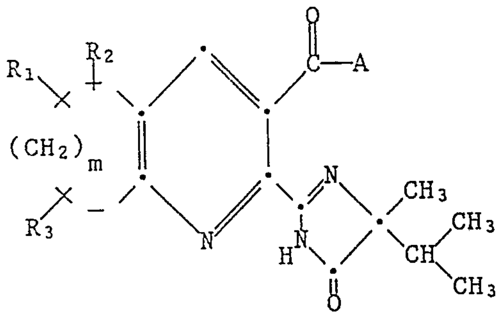

(Ia)

worin A für OH steht und die Reste X, $R_1$, $R_2$, $R_3$ und m wie zuvor definiert sind, umsetzt, oder

b) eine Verbindung der Formel Ia

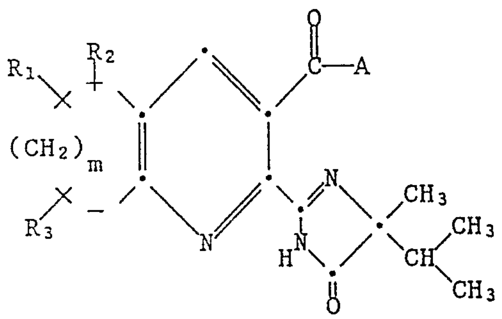

(Ia)

worin $R_1$, $R_2$, $R_3$ und m wie zuvor definiert sind und A für OH steht in Gegenwart eines wasserbindenden bzw. wasserabscheidenden Mittels zu einer Verbindung der Formel Ib

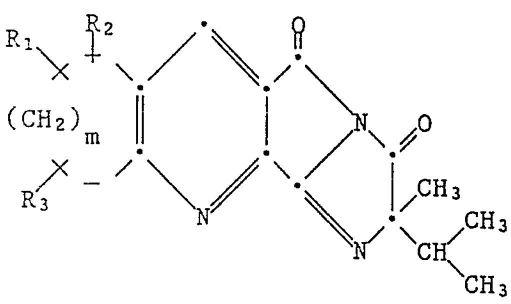

(Ib)

worin $R_1$, $R_2$, $R_3$ und m wie zuvor definiert sind, cyclisiert, oder

c) ein Säureanhydrid der Formel IV

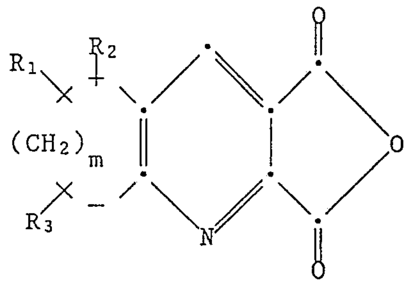

(IV)

worin $R_1$, $R_2$, $R_3$ und m wie zuvor definiert sind, mit 2-Amino-2,3-dimethylbuttersäureamid der Formel III

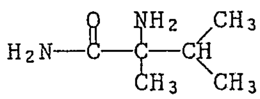

(III)

zu einem Imid der Formel V

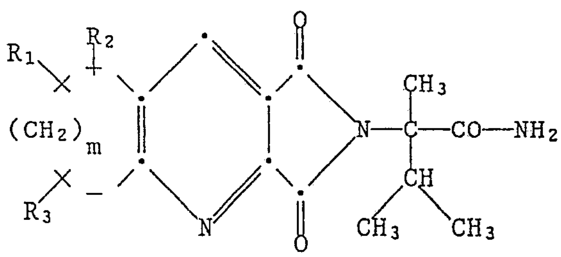

(V)

umsetzt und das so erhältliche Imid der Formel V in Gegenwart einer Base zu einer Verbindung der Formel Ic

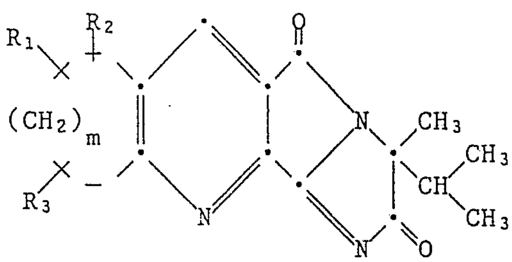

(Ic)

worin $R_1$, $R_2$, $R_3$ und m wie zuvor definiert sind, cyclisiert, oder

d) eine Verbindung der Formel Ib

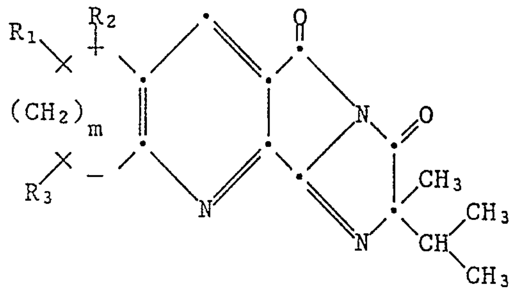

(Ib)

worin $R_1$, $R_2$, $R_3$ und m wie zuvor definiert sind zu einer Verbindung der Formel Ic

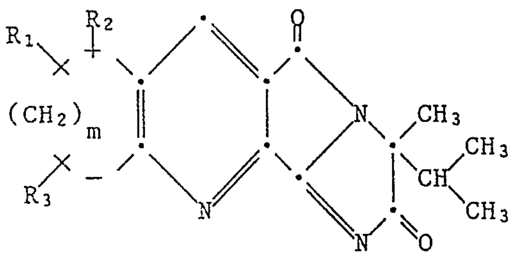

(Ic)

worin $R_1$, $R_2$, $R_3$ und m wie zuvor definiert sind, isomerisiert, oder

e) eine Verbindung der Formel Ib

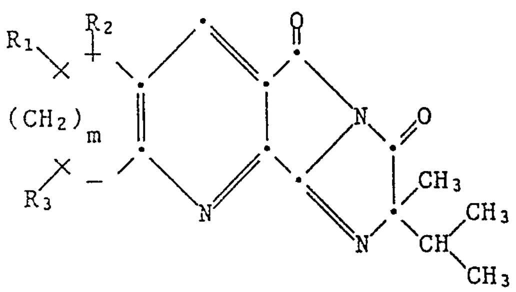

(Ib)

oder eine Verbindung der Formel Ic

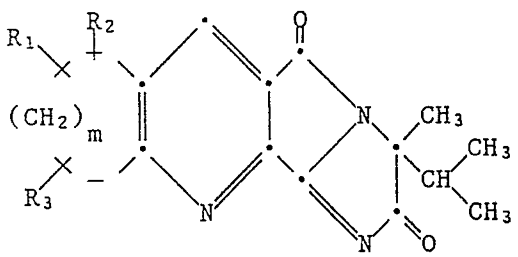

(Ic)

in welchen die Reste $R_1$, $R_2$, $R_3$ und m wie zuvor definiert sind, mit einem Alkohol der Formel VI

H A VI,

worin A für $C_1$-$C_4$-Alkoxy steht, gewünschtenfalls in Gegenwart einer Säure oder Base zu einer Verbindung der Formel Ia

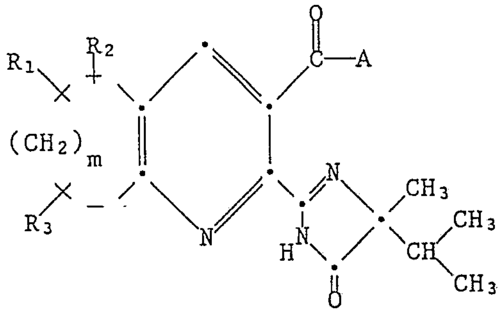

(Ia)

worin $R_1$, $R_2$, $R_3$ und m wie zuvor definiert sind und A für $C_1$-$C_4$-Alkoxy steht, umsetzt, oder

f) einen Diester der Formel II

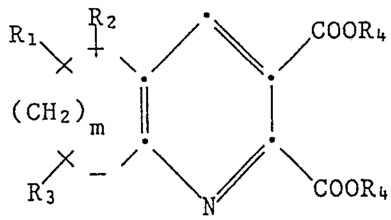

(II)

in dem $R_1$, $R_2$, $R_3$ und m wie zuvor definiert sind und $R_4$ für $C_1$-$C_4$-Alkyl steht, unter nicht hydrolisierenden Bedingungen und in Gegenwart einer Base mit 2-Amino-2,3-dimethylbuttersäureamid zu einer Verbindung der Formel Ia

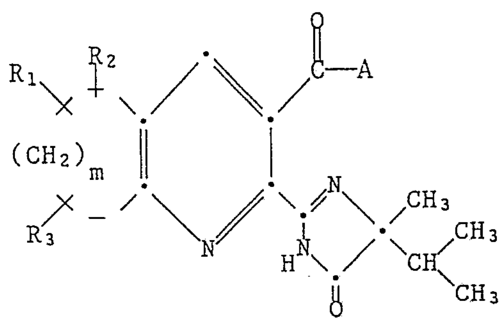

(Ia)

worin $R_1$, $R_2$, $R_3$ und m wie zuvor definiert sind und A für $C_1$-$C_4$-Alkoxy steht, umsetzt, oder

g) eine Verbindung der Formel Ia

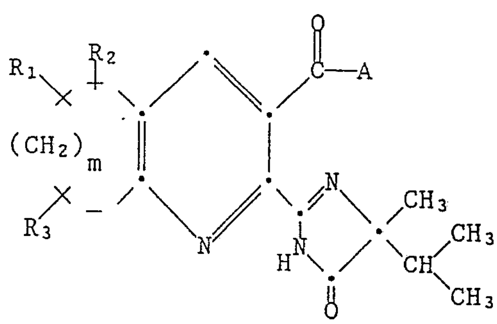

(Ia)

in der die Reste $R_1$, $R_2$, $R_3$ und m wie zuvor definiert sind und A für OH oder $C_1$-$C_4$-Alkoxy steht mit

einer äquivalenten Menge einer Verbindung der Formel VII
$OH^{\ominus}M^{\oplus}$ (VII)
in der $M^{\oplus}$ für einen Kationenäquivalent eines Alkali- oder Erdalkalimetalls oder eine quaternäre stickstoffhaltige Base steht, zu einem Salz der Formel Ia′

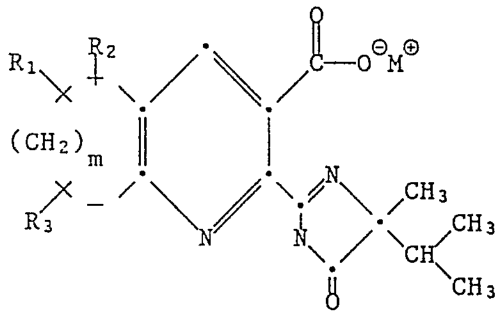

(Ia')

worin $R_1$, $R_2$, $R_3$ und m wie zuvor definiert sind und $M^{\oplus}$ für ein Kationenäquivalent eines Alkali- oder Erdalkalimetalls oder einer stickstoffhaltigen Base steht, umsetzt, oder

h) eine Verbindung der Formel Ia, in der A für OH steht mit einem Alkali-oder Erdalkalimetall, einem Alkali oder Erdalkalisalz oder der stickstoffhaltigen Base direkt zu einem Salz der Formel Ia′

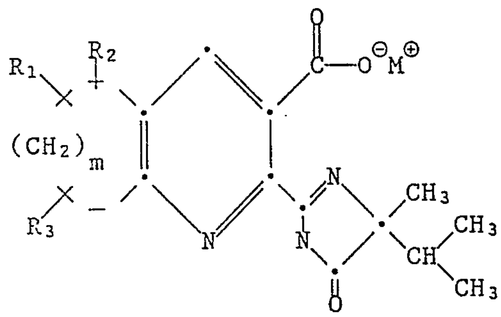

(Ia')

worin $R_1$, $R_2$, $R_3$ und m wie zuvor definiert sind und $M^{\oplus}$ für ein Kationenäquivalent eines Alkali- oder Erdalkalimetalls oder einer stickstoffhaltigen Base steht, umsetzt.

2. Verfahren gemäss Anspruch 1 zur Herstellung von Verbindungen der Formel I, worin
$R_1$ $C_1$-$C_3$-Alkyl;
$R_2$ und $R_3$ unabhängig voneinander Wasserstoff oder $C_1$-$C_3$-Alkyl;
$R_1$ und $R_2$ ausserdem gemeinsam einen geminal gebundenen $-(CH_2)_n-$Ring;
m 3 oder 4;
n 2, 4 oder 5;
A OH, $O^{\ominus}M^{\oplus}$ oder $C_1$-$C_3$-Alkoxy;
B ein Imidazolon der Formel

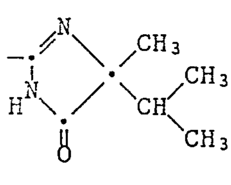

;

oder
A und B gemeinsam ein anelliertes Imidazolon der Formel

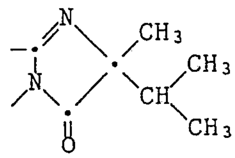

bedeutet.

3. Verbindung gemäss Anspruch 1 oder 2 zur Herstellung von Verbindung der Formel I, worin $R_1$ Methyl oder Ethyl; $R_2$ und $R_3$ unabhängig voneinander Wasserstoff; Methyl oder Ethyl; $R_1$ und $R_2$ ausserdem gemeinsam einen geminal gebundenen $-(CH_2)_n-$Ring mit $n = 2$ oder 4 bedeutet und die Reste m, A und B wie zuvor definiert sind.

4. Verfahren gemäss Anspruch 1 zur Herstellung von
7H-5,6-Dihydro-2-(5-isopropyl-5-methyl-1H-imidazol-4-on-2-yl)-5-methyl-1-pyridin-3-carbonsäure,
7H-5,6-Dihydro-2-(5-isopropyl-5-methyl-1H-imidazol-4-on-2-yl)-6-methyl-1-pyrindin-3-carbonsäure,
2-(5-Isopropyl-5-methyl-1H-imidazol-4-on-2-yl)-5-methyl-5,6,7,8-tetrahydrochinolin-3-carbonsäure,
2-(5-Isopropyl-5-methyl-1H-imidazol-4-on-2-yl)-7-methyl-5,6,7,8-tetrahydrochinolin-3-carbonsäure,
2-(5-Isopropyl-5-methyl-1H-imidazol-4-on-2-yl)-8-methyl-5,6,7,8-tetrahydrochinolin-3-carbonsäure,
2-(5-Isopropyl-5-methyl-1H-imidazol-4-on-2-yl)-5,6,7,8-tetrahydro-5,7,7-trimethylchinolin-3-carbonsäure,
2-(5-Isopropyl-5-methyl-1H-imidazol-4-on-2-yl)-6-methyl-5,6,7,8-tetrahydrochinolin-3-carbonsäure,
6,6-Dimethyl-2-(5-isopropyl-5-methyl-1H-imidazol-4-on-2-yl)-5,6,7,8-tetrahydrochinolin-3-carbonsäure,
6,6-Dimethyl-2-(5-isopropyl-5-methyl-1H-imidazol-4-on-2-yl)-3-methoxycarbonyl-5,6,7,8-tetrahydrochinolin,
2-(5-Isopropyl-5-methyl-1H-imidazol-4-on-2-yl)-3-methoxycarbonyl-7-methyl-5,6,7,8-tetrahydrochinolin,
2,7-Dimethyl-2-isopropyl-7,8,9,10-tetrahydro-3H,5H-imidazo[1′,2′:1,2]pyrrolo[3,4-b]chinolin-3,5-dion,
2,8-Dimethyl-2-isopropyl-7,8,9,10-tetrahydro-3H,5H-imidazo[1′,2′:1,2]pyrrolo[3,4-b]chinolin-3,5-dion
2-Isopropyl-7,8,9,10-tetrahydro-2,8,8-trimethyl-3H,5H-imidazo[1′,2′:1,2]pyrrolo[3,4-b]chinolin-3,5-dion
oder
2,9-Dimethyl-2-isopropyl-7,8,9,10-tetrahydro-3H,5H-imidazo[1′,2′:1,2]pyrrolo[3,4-b]chinolin-3,5-dion.

5. Verfahren zur Bekämpfung unerwünschten Pflanzenwuchses, dadurch gekennzeichnet, dass man eine Verbindung der Formel I gemäss einem der Ansprüche 1 bis 4 auf die zu bekämpfende Pflanze oder deren Lebensraum einwirken lässt.

6. Verfahren zur Beeinflussung des vegetativen Wachstums von Pflanzen, dadurch gekennzeichnet, dass man eine pflanzenregulatorisch wirksame Menge einer Verbindung der Formel I gemäss einem der Ansprüche 1 bis 4 auf die Pflanze oder deren Lebensraum einwirken lässt.

7. Verfahren zur Herstellung eines herbiziden oder wuchsregulatorischen Mittels, dadurch gekennzeichnet, dass man eine Verbindung der Formel I gemäss einem der Ansprüche 1 bis 4 mit Hilfs- und/oder Trägerstoffen mischt.

8. Verfahren zur Beizung von Saatgut, dadurch gekennzeichnet, dass man das Saatgut mit einer herbizid oder pflanzenwuchsregulatorisch wirksamen Menge einer Verbindung der Formel I gemäss einem der Ansprüche 1 bis 4 behandelt.

9. Verfahren zur Herstellung der Zwischenprodukte der Formeln II, XII, IV oder V gemäss Anspruch 1, dadurch gekennzeichnet, dass man

a) ein Enamin der Formel VIII

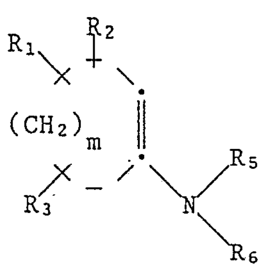

(VIII)

worin $R_5$ und $R_6$ unabhängig voneinander für $C_1$-$C_4$-Alkyl oder $R_5$ und $R_6$ gemeinsam eine, gewünschtenfalls einmal durch Sauerstoff oder N-$R_7$ (mit $R_7$ = Wasserstoff oder $C_1$-$C_4$-Alkyl) unterbrochene $-(CH_2)_p-$ Kette bedeutet mit p = 4-6 und $R_1$, $R_2$, $R_3$ und m wie zuvor definiert sind, mit einem Diester der Formel IX

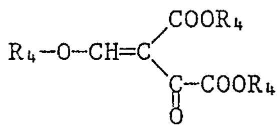

(IX),

worin $R_4$ für $C_1$-$C_4$-Alkyl steht umsetzt, und das aus VIII und IX erhältliche Monokondensationsprodukt in einem zweiten Reaktionsschritt mit Ammoniak zu einem Diester der Formel II

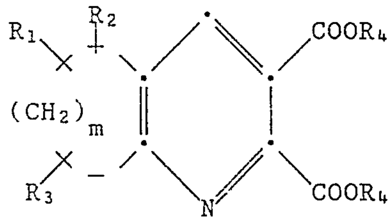

(II)

worin
$R_1$ $C_1$-$C_4$-Alkyl;
$R_2$ und $R_3$ unabhängig voneinander Wasserstoff; oder $C_1$-$C_4$-Alkyl;
$R_1$ und $R_2$ ausserdem gemeinsam einen geminal gebundenen $-(CH_2)_n-$Ring;
m 3 oder 4;
n 2 bis 5; und
$R_4$ $C_1$-$C_4$-Alkyl bedeutet, cyclisiert, oder
b) im β-Hydroxyketon der Formel X

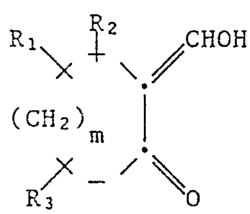

(X)

worin $R_1$, $R_2$, $R_3$ und m wie zuvor definiert sind mit einem Aminofumarat XI

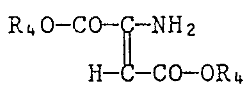

(XI)

worin $R_4$ wie zuvor definiert ist zu einem Diester der Formel II

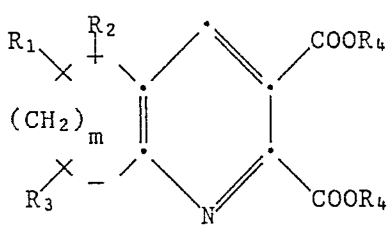

(II)

worin
$R_1$ $C_1$-$C_4$-Alkyl;
$R_2$ und $R_3$ unabhängig voneinander Wasserstoff; oder $C_1$-$C_4$-Alkyl;
$R_1$ und $R_2$ ausserdem gemeinsam einen geminal gebundenen $-(CH_2)_n-$Ring;
m 3 oder 4;
n 2 bis 5; und
$R_4$ $C_1$-$C_4$-Alkyl bedeutet, kondensiert, oder
c) einen Ester der Formel II

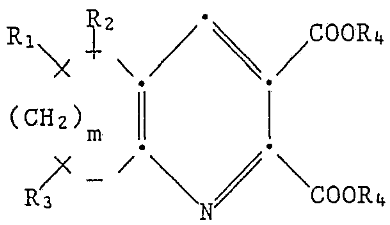

(II),

worin $R_1$, $R_2$, $R_3$ und m wie zuvor definiert sind und $R_4$ $C_1$-$C_4$-Alkyl bedeutet zu einer Dicarbonsäure der Formel XII

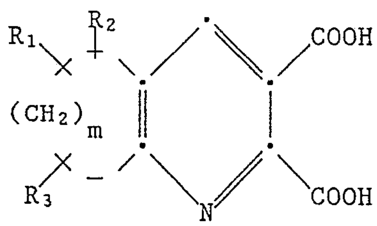

(XII),

worin
$R_1$ $C_1$-$C_4$-Alkyl;
$R_2$ und $R_3$ unabhängig voneinander Wasserstoff; oder $C_1$-$C_4$-Alkyl;
$R_1$ und $R_2$ ausserdem gemeinsam einen geminal gebundenen $-(CH_2)_n-$Ring;
m 3 oder 4; und
n 2 bis 5; bedeutet, verseift, oder
d) einen Ester der Formel II

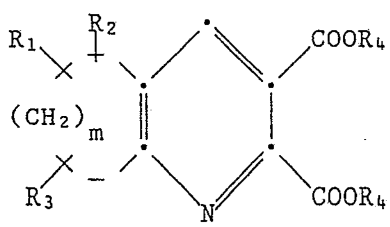

(II),

worin $R_1$, $R_2$, $R_3$ und m wie zuvor definiert sind und $R_4$ $C_1$-$C_4$-Alkyl bedeutet, zu einer Dicarbonsäure der Formel XII

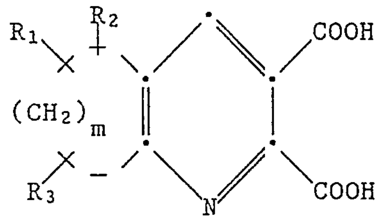

(XII),

worin $R_1$, $R_2$, $R_3$ und m wie zuvor definiert sind, verseift und anschliessend die so erhältliche Dicarbonsäure XII mit wasserabspaltenden Mitteln zu dem Anhydrid IV

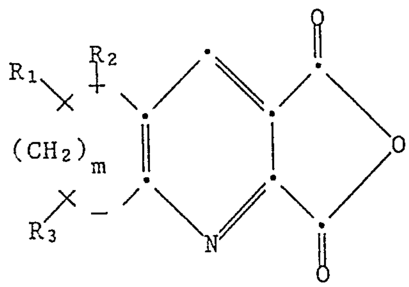

(IV),

worin
$R_1$ $C_1$-$C_4$-Alkyl;
$R_2$ und $R_3$ unabhängig voneinander Wasserstoff; oder $C_1$-$C_4$-Alkyl;
$R_1$ und $R_2$ ausserdem gemeinsam einen geminal gebundenen $-(CH_2)_n-$Ring;
m 3 oder 4; und
n 2 bis 5; bedeutet, cyclisiert, oder

e) ein Anhydrid der Formel IV

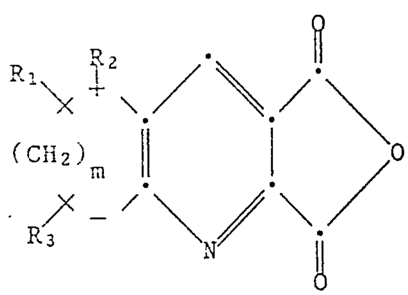

(IV),

worin $R_1$, $R_2$, $R_3$ und m wie zuvor definiert sind, mit 2-Amino-2,3-dimethylbuttersäureamid III

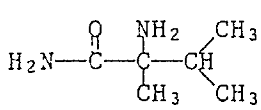

(III)

zu einem Imid der Formel V

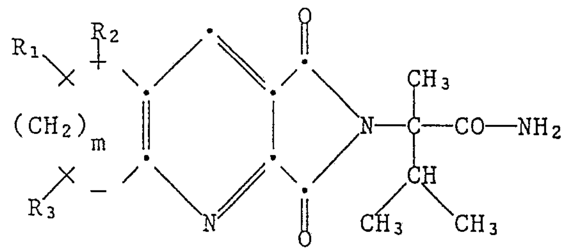

(V)

worin

$R_1$ $C_1$-$C_4$-Alkyl;

$R_2$ und $R_3$ unabhängig voneinander Wasserstoff; oder $C_1$-$C_4$-Alkyl;

$R_1$ und $R_2$ ausserdem gemeinsam einen geminal gebundenen $-(CH_2)_n-$Ring;

m 3 oder 4; und

n 2 bis 5; bedeutet, umsetzt.

Europäisches Patentamt

# EUROPÄISCHER RECHERCHENBERICHT

Nummer der Anmeldung

EP 89 81 0031

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS Band 105, Nr. 23, 9. Dezember 1986, Seite 584, Spalte 2, Zusammenfassung Nr. 208767z, Columbus, Ohio, US; J.L. JOHNSON et al.:"Quinolinic acid esters"; & BR - A - 8502364 (AMERICAN CYANAMID CO.) 21.01.1986 * Zusammenfassung, in Zusammenhang mit CHEMICAL SUBSTANCE INDEX, Band 105, Teil 9, Juli-Dezember 1986, Seite 7453CS, Spalte 2 * | 6,7 | C 07 D 401/04<br>C 07 D 471/14<br>C 07 D 215/54<br>C 07 D 221/04<br>C 07 D 491/04<br>C 07 D 471/04<br>A 01 N 43/50<br>A 01 N 43/90 //<br>(C 07 D 471/14<br>C 07 D 235:00<br>C 07 D 221:00<br>C 07 D 209 00 )<br>(C 07 D 491/04<br>C 07 D 307:00<br>C 07 D 221:00 )<br>(C 07 D 471/04<br>C 07 D 221:00<br>-/- |
| Y | idem<br>--- | 1-5,8-10 | |
| Y,D | GB-A-2 174 395 (AMERICAN CYANAMID CO.) * Seite 165, Zeilen 42,43; Ansprüche 1,2,6,7,9,43,44,46,47,59-67,70-76 *<br>--- | 1-5,8-10 | |
| A,D | EP-A-0 212 200 (NISSAN CHEMICAL INDUSTRIES LTD) * Herstellungsbeispiel 9; Beispiel 29, Ansprüche 1-4,9-12,14 *<br>----- | 1,5-10 | |

| RECHERCHIERTE SACHGEBIETE (Int. Cl.4) |
|---|
| C 07 D 401/00 |
| C 07 D 471/00 |
| C 07 D 215/00 |
| C 07 D 221/00 |
| C 07 D 491/00 |
| A 01 N 43/00 |

Der vorliegende Recherchenbericht wurde für alle Patentansprüche erstellt

| Recherchenort | Abschlußdatum der Recherche | Prüfer |
|---|---|---|
| BERLIN | 26-04-1989 | VAN AMSTERDAM L.J.P. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 89 81 0031

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| | | | C 07 D 209:00 ) |
| | | | RECHERCHIERTE SACHGEBIETE (Int. Cl.4) |

Der vorliegende Recherchenbericht wurde für alle Patentansprüche erstellt

| Recherchenort | Abschlußdatum der Recherche | Prüfer |
|---|---|---|
| BERLIN | 26-04-1989 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)